# EUROPEAN PATENT SPECIFICATION

(11) **EP 0 731 088 B1**
(45) Date of publication and mention of the grant of the patent: **04.10.2000**
(21) Application number: 96107359.0
(22) Date of filing: 18.11.1991
(51) Int. Cl.: C07C 271/22, C07D 215/48, A61K 31/325, A61K 31/47

(54) **Retroviral protease inhibitors**
Inhibitoren retroviraler Proteasen
Inhibiteurs de protéases rétrovirales

(30) Priority: 19.11.1990 US 615210; 14.11.1991 US 789643
(43) Date of publication of application: 11.09.1996
(62) Divisional of application: 92901691.3
(73) Proprietor: MONSANTO COMPANY, St. Louis, Missouri 63167 (US)
(72) Inventor: Reed, Kathryn Lea, North Carolina 27613 (US); Talley, John Jeffrey, Brentwood, Missouri 63144 (US)
(74) Representative: Bosch, Henry

(56) References cited:
- EP-A- 0 172 347
- EP-A- 0 223 437
- FR-A- 2 620 451
- JOURNAL OF MEDICINAL CHEMISTRY, vol. 30, no. 7, July 1987, WASHINGTON, DC, US, pages 1224-1228, XP002027217 S.H. ROSENBERG, ET AL.: "Novel renin inhibitors containing analogues of statine retro-inverted at the C-termini: specificity at the P2 histidine site"

## Description

### 1. Field of the Invention

The present invention relates to retroviral protease inhibitors and, more particularly, relates to novel compounds and the use of the compounds for the manufacture of a medicament for inhibiting retroviral proteases. This invention, in particular, relates to hydroxyethylamine protease inhibitor compounds, and the use of the compounds for the manufacture of a medicament for inhibiting retroviral proteases such as human immunodeficiency virus (HIV) protease and for treating a retroviral infection, e.g., an HIV infection.

### 2. Related Art

During the replication cycle of retroviruses, gag and gag-pol gene products are translated as proteins. These proteins are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse transcriptase and retroviral protease. It has been shown that correct processing of the precursor proteins by the retroviral protease is necessary for assembly of infectious virons. For example, it has been shown that frameshift mutations in the protease region of the pol gene of HIV prevents processing of the gag precursor protein. It has also been shown through site-directed mutagenesis of an aspartic acid residue in the HIV protease that processing of the gag precursor protein is prevented. Thus, attempts have been made to inhibit viral replication by inhibiting the action of retroviral proteases.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit replication of structural proteins and, more importantly, the retroviral protease itself. In this manner, retroviral replication proteases can be effectively inhibited.

Several classes of mimetic compounds have been proposed, particularly for inhibition of proteases, such as for inhibition of HIV protease. Such mimetics include hydroxyethylamine isosteres and reduced amide isosteres. See, for example, EP O 346 847; EP O 342,541; Roberts et al, "Rational Design of Peptide-Based Proteinase Inhibitors, "Science, 248, 358 (1990); and Erickson et al, "Design Activity, and 2.8A Crystal Structure of a C₂ Symmetric Inhibitor Complexed to HIV-1 Protease," Science, 249, 527 (1990).

Several classes of mimetic compounds are known to be useful as inhibitors of the proteolytic enzyme renin. See, for example, U.S. No. 4,599,198; U.K. 2,184,730; G.B. 2,209,752; EP O 264 795; G.B. 2,200,115 and U.S. SIR H725. However, it is known that, although renin and HIV proteases are both classified as aspartyl proteases, compounds which are effective renin inhibitors generally cannot be predicted to be effective HIV protease inhibitors.

### BRIEF DESCRIPTION OF THE INVENTION

The present invention is directed to virus inhibiting compounds and compositions. More particularly, the present invention is directed to retroviral protease inhibiting compounds and the use of the compounds for the manufacture of a medicament for inhibiting retroviral proteases.

### DETAILED DESCRIPTION OF THE INVENTION

In accordance with the present invention, there is provided a retroviral protease inhibiting compound of the formula: 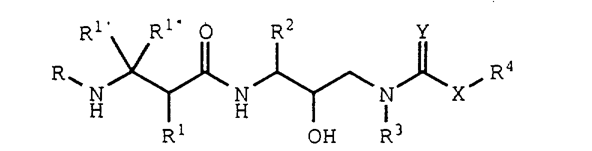 or a pharmaceutically acceptable salt thereof, wherein the stereochemistry about the hydroxy group is (R) ;
Y represents O or S;
R² represents alkyl, cycloalkylalkyl or aralkyl radicals, which radicals are optionally substituted with halogen, -OR⁹ or -SR⁹ radicals, wherein R⁹ represents alkyl radical; and
R¹ represents methyl radical and R^{1'} and R^{1"} independently represent hydrogen or radical as defined for R¹; and R represents alkanoyl, arylalkanoyl, aryloxyalkanoyl, arylalkoxycarbonyl, heteroaroyl or alkylaminocarbonyl radicals; or
R¹ and R^{1'} are both hydrogen and R^{1"} represents -CH₂SO₂NH₂, alkyl of 1 to 4 carbon atoms or cycloalkyl radicals or side chain of the amino acid asparagine, S-methyl cysteine or the sulfone or sulfoxide derivatives thereof, histidine, norleucine, glutamine, glycine, allo-isoleucine, alanine, threonine, isoleucine, leucine, tert-leucine, phenylalanine, ornithine, allo-threonine or valine; and R represents arylalkoxycarbonyl or heteroaroyl radicals; and
X represents O or C(R⁵)(R¹⁷), wherein R¹⁷ represents hydrogen or alkyl radicals; and
R³ and R⁴ independently represent alkyl, cycloalkyl, cycloalkylalkyl, aryl or aralkyi radicals; and R⁵ represents hydrogen radical; or
R³ represents alkyl radical of 2 to 5 carbon atoms; and
R⁴ and R⁵ together with the carbon atom to which they are attached represent a 5 or 6-membered cycloalkyl radical optionally substituted with an alkyl radical of 1 to 3 carbon atoms; and
wherein alkyl, alone or in combination, is a straight-chain or branched-chain alkyl radical containing from 1 to 8 carbon atoms; cycloalkyl, alone or in combination, is an cyclic alkyl radical containing from 3 to 8 carbon atoms; aryl, alone or in combination, is an unsubstituted phenyl or naphthyl radical, or phenyl or naphthyl radical substituted with a C₁-C₈ alkyl, C₁-C₈ alkoxy, halogen, hydroxy or amino radical; heterocycloalkyl, alone or in combination, is pyrrolidinyl, piperidinyl, piperazinyl, morpholinyl, thiamorpholinyl, tetrahydroquinolinyl or tetrahydroisoquinolinyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido; and heteroaryl, alone or in combination, is pyrrolyl, imidazolyl, pyrazolyl, pyridyl, pyrazinyl, pyrimidinyl, furyl, thienyl, triazolyl, oxazolyl, thiazolyl, indolyl, quinolinyl, isoquinolinyl, quinoxalinyl, β-carbolinyl, benzofuranyl or benzimidazolyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido.

Preferably, R³ represents radicals as defined above which contain no α-branching, e.g., as in an isopropyl radical or a t-butyl radical. The preferred radicals are those which contain a -CH₂- moiety between the nitrogen of the urea and the remaining portion of the radical. Such preferred groups include, but are not limited to, benzyl, isobutyl, n-butyl, isoamyl and cyclohexylmethyl.

As utilized herein, the term "alkyl", alone or in combination, means a straight-chain or branched-chain alkyl radical containing from 1 to 10, preferably from 1 to 8, carbon atoms. Examples of such radicals include methyl, ethyl, n-propyl, isopropyl, n-butyl, isobutyl, sec-butyl, tert-butyl, pentyl, isoamyl, hexyl and octyl. The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, and tert-butoxy. The term "cycloalkyl" means an alkyl radical which contains from 3 to 8 carbon atoms and is cyclic. The term "cycloalkylalkyl" means an alkyl radical as defined above which is substituted by a cycloalkyl radical containing from 3 to 8, preferably from 3 to 6, carbon atoms. Examples of such cycloalkyl radicals include cyclopropyl, cyclobutyl, cyclopentyl and cyclohexyl. The term "aryl", alone or in combination, means a phenyl or naphthyl radical which optionally carries one or more substituents selected from alkyl, alkoxy, halogen, hydroxy, amino and the like, such as phenyl, p-tolyl, 4-methoxyphenyl, 4-(tert-butoxy)phenyl, 4-fluorophenyl, 4-chlorophenyl, 4-hydroxyphenyl, 1-naphthyl and 2-naphthyl. The term "aralkyl", alone or in combination, means an alkyl radical as defined above in which one hydrogen atom is replaced by an aryl radical as defined above, such as benzyl and 2-phenylethyl. The term "aralkoxy carbonyl", alone or in combination, means a radical of the formula -C(O)-O-aralkyl in which the term "aralkyl" has the significance given above. An example of an aralkoxycarbonyl radical is benzyloxycarbonyl. The term "aryloxy" means a radical of the formula aryl-o- in which the term aryl has the significance given above. The term "alkanoyl", alone or in combination, means an acyl radical derived from an alkanecarboxylic acid, examples of which include acetyl, propionyl, butyryl, valeryl and 4-methylvaleryl. The term "cycloalkylcarbonyl" means an acyl group derived from a monocyclic or bridged cycloalkanecarboxylic acid such as cyclopropanecarbonyl, cyclohexanecarbonyl and adamantanecarbonyl, or from a benz-fused monocyclic cycloalkanecarboxylic acid which is optionally substituted by, for example, alkanoylamino, such as 1,2,3,4-tetrahydro-2-naphthoy 1,2 -acetamido-1,2,3,4-tetrahydro-2-naphthoyl. The term "aralkanoyl" means an acyl radical derived from an aryl-substituted alkanecarboxylic acid such as phenylacetyl, 3-phenylpropionyl (hydrocinnamoyl), 4-phenylbutyryl, (2-naphthyl)acetyl, 4-chlorohydrocinnamoyl, 4-aminohydroinnamoyl and 4-methoxyhydrocinnamoyl.

The term "aroyl" means an acyl radical derived from an aromatic carboxylic acid. Examples of such radicals include aromatic carboxylic acids, an optionally substituted benzoic or naphthoic acid such as benzoyl, 4-chlorobenzoyl, 4-carboxybenzoyl, 4-(benzyloxycarbonyl)benzoyl, 1-naphthoyl, 2-naphthoyl, 6-carboxy-2 naphthoyl, 6-(benzyloxycarbonyl)-2-naphthoyl, 3-benzyloxy-2-naphthoyl, 3-hydroxy-2-naphthoyl and 3-(benzyloxyformamido)-2-naphthoyl. The heterocyclyl or heterocycloalkyl portion of a heterocyclylcarbonyl, heterocyclyloxycarbonyl, heterocyclylalkoxycarbonyl, or heterocyclyalkyl group is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy, oxo, and the like, and/or on a secondary nitrogen atom (i.e., -NH-) by alkyl, aralkoxycarbonyl, alkanoyl, phenyl or phenylalkyl or on a tertiary nitrogen atom (i.e. = N-) by oxido and which is attached via a carbon atom. The heteroaryl portion of a heteroaroyl, heteroaryloxycarbonyl, or a heteroaralkoxy carbonyl group is an aromatic monocyclic, bicyclic, or tricyclic heterocycle which contains the hetero atoms and is optionally substituted as defined above with respect to the definition of heterocyclyl. Examples of such heterocyclyl and heteroaryl groups are pyrrolidinyl, piperidinyl, piperazinyl, morpholinyl, thiamorpholinyl, pyrrolyl, imidazolyl (e.g., imidazol 4-yl, 1-benzyloxycarbonylimidazol-4-yl, etc.), pyrazolyl, pyridyl, pyrazinyl, pyrimidinyl, furyl, thienyl, triazolyl, oxazolyl, thiazolyl, indolyl (e.g., 2-indolyl), quinolinyl, (e.g., 2-quinolinyl, 3-quinolinyl, 1-oxido-2-quinolinyl), isoquinolinyl (e.g., 1-isoquinolinyl, 3-isoquinolinyl), tetrahydroquinolinyl (e.g., 1,2,3,4-tetrahydro-2-quinolyl), 1,2,3,4-tetrahydroisoquinolinyl (e.g., 1,2,3,4-tetrahydro-1-oxo-isoquinolinyl, etc.), quinoxalinyl, β-carbolinyl, benzofurancarbonyl, and benzimidazolyl radicals. The term "cycloalkylalkoxycarbonyl" means an acyl group derived from a cycloalkylalkoxycarboxylic acid of the formula cycloalkylalkyl-O-COOH wherein cycloalkylalkyl has the significance given above. The term "aryloxyalkanoyl" means an acyl radical of the formula aryl-O-alkanoyl wherein aryl and alkanoyl have the significance given above. The term "heterocyclyloxycarbonyl" means an acyl group derived from heterocyclyl-O-COOH wherein heterocyclyl is as defined above. The term "heterocyclylalkanoyl" is an acyl radical derived from a heterocyclyl-substituted alkane carboxylic acid wherein heterocyclyl has the significance given above. The term "heterocyclylalkoxycarbonyl" means an acyl radical derived from a heterocyclyl-substituted alkane-O-COOH wherein heterocyclyl has the significance given above. The term "heteroaryloxycarbonyl" means an acyl radical derived from a carboxylic acid represented by heteroaryl-O-COOH wherein heteroaryl has the significance given above. The term "aminoalkanoyl" means an acyl group derived from an amino-substituted alkanecarboxylic acid wherein the amino group can be a primary, secondary or tertiary amino group containing substituents selected from hydrogen, and alkyl, aryl, aralkyl, cycloalkyl and cycloalkylalkyl radicals. The term "halogen" means fluorine, chlorine, bromine or iodine. The term "leaving group" generally refers to groups readily displaceable by a nucleophile, such as an amine, a thiol or an alcohol nucleophile. Such leaving groups are well known and include carboxylates, N-hydroxysuccinimide, N-hydroxybenzotriazole, halides, triflates, tosylates -OR and -SR. Preferred leaving groups are indicated herein where appropriate.

### Preparation of Compounds of Invention

The compounds of the present invention represented by Formula II above can be prepared utilizing the following general procedure. An N-protected chloroketone derivative of an amino acid having the formula: 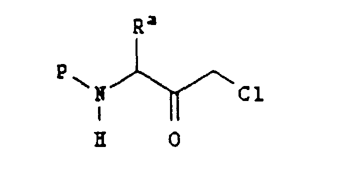 wherein P represents an amino protecting group, and R² is as defined above, is reduced to the corresponding alcohol utilizing an appropriate reducing agent. Suitable amino protecting groups are well known in the art and include carbobenzoxy, butyryl, t-butoxycarbonyl, acetyl and benzoyl. A preferred amino protecting group is carbobenzoxy. A preferred N-protected chloroketone is N-benzyloxycarbonyl-L-phenylalanine chloromethyl ketone. A preferred reducing agent is sodium borohydride. The reduction reaction is conducted at a temperature of from -10°C to 25°C, preferably at 0°C, in a suitable solvent system such as, for example, tetrahydrofuran. The N-protected chloroketones are commercially available from Bachem, Inc., Torrance, California. Alternatively, the chloroketones can be prepared by the procedure set forth in S. J. Fittkau, J. Prakt. Chem., 315, 1037 (1973), and subsequently N-protected utilizing procedures which are well known in the art.

The resulting alcohol is then reacted, preferably at room temperature, with a suitable base in a suitable solvent system to produce an N-protected amino epoxide of the formula: 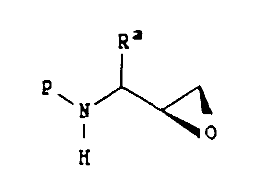 wherein P and R² are as defined above. Suitable solvent systems for preparing the amino epoxide include ethanol, methanol, isopropanol, tetrahydrofuran, dioxane, and mixtures thereof. Suitable bases for producing the epoxide from the reduced chloroketone include potassium hydroxide, sodium hydroxide, potassium t-butoxide, DBU and the like. A preferred base is potassium hydroxide.

The amino epoxide is then reacted, in a suitable solvent system, with an equal amount, or preferably an excess of, a desired amine of the formula:

R³NH₂

wherein R³ is hydrogen or is as defined above. The reaction can be conducted over a wide range of temperatures, e.g., from 10°C to 100°C, but is preferably, but not necessarily, conducted at a temperature at which the solvent begins to reflux. Suitable solvent systems include those wherein the solvent is an alcohol, such as methanol, ethanol, isopropanol, ethers such as tetrahydrofuran, dioxane and toluene, N,N-dimethylformamide, dimethyl sulfoxide, and mixtures thereof. A preferred solvent is isopropanol. Exemplary amines corresponding to the formula R³NH₂ include benzyl amine, isobutylamine, n-butyl amine, isopentyl amine, isoamylamine, cyclohexanemethyl amine and naphthylene methyl amine. The resulting product is a 3-(N-protected amino)-3-(R²)-1-(NHR³)-propan-2-ol derivative (hereinafter referred to as an amino alcohol) can be represented by the formula: 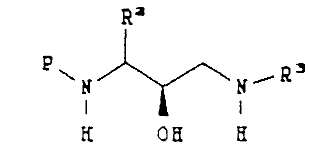 wherein P, R² and R³ are as described above.

Where X is either O or C, the appropriate analogs can be prepared by reacting the above described amino alcohol with an acid chloride or anhydride to form the analog wherein X is C, or with a chloroformate or pyrocarbonate where X is O. Procedures for reacting these compounds with an amine are well known in the art. Examples of such compounds include t-butylacetyl chloride, acetic anhydride, t-butyl pyrocarbonate, and butyl chloroformate. These analogs can be represented by the formulas:

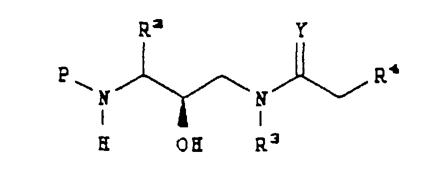

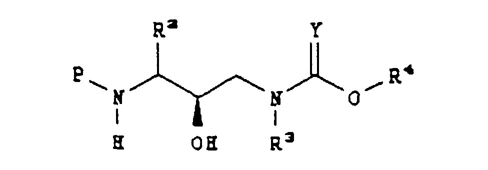

The derivative of the amino alcohol and the corresponding sulfur analog can be represented by the formula:

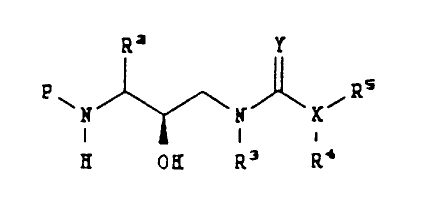

Following preparation of such derivatives, the amino protecting group P is removed under conditions which will not affect the remaining portion of the molecule. These methods are well known in the art and include acid hydrolysis and hydrogenolysis. A preferred method involves removal of the protecting group, e.g., removal of a carbobenzoxy group, by hydrogenolysis utilizing palladium on carbon in a suitable solvent system such as an alcohol, acetic acid, or mixtures thereof. Where the protecting group is a t-butoxycarbonyl group, it can be removed utilizing an inorganic or organic acid, e.g., HCl or trifluoroacetic acid, in a suitable solvent system, e.g., dioxane or methylene chloride. The resulting product is the amine salt derivative. Following neutralization of the salt, the amine is then reacted with an amino acid or corresponding derivative thereof represented by the formula (PN[CR^{1'}R^{1"}]ₜCH(R¹)COOH) wherein t, R¹, R^{1'} and R^{1"} are as defined above, to produce the antiviral compounds of the present invention having the formula: 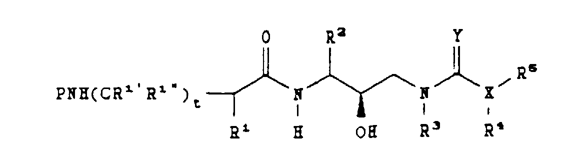 wherein t, X, P, R¹, R^{1'}, R^{1''}, R², R³, R⁴, R⁵ and Y are as defined above. Preferred protecting groups in this instance are a benzyloxycarbonyl group or a t-butoxycarbonyl group. Where the amine is reacted with a derivative of an amino acid, e.g., when t=1 and R^{1'} and R^{1"} are both H, so that the amino acid is a β-amino acid, such β-amino acids can be prepared according to the procedure set forth in a copending application, U. S. Serial No. 07/345,808. Where t is 1, one of R^{1'} and R^{1"} is H and R¹ is hydrogen so that the amino acid is a homoβ-amino acid, such homo-β-amino acids can be prepared by the same procedure. Where t is O and R¹ is alkyl, cycloalkyl, -CH₂SO₂NH₂ or an amino acid side chain, such materials are well known and many are commercially available from Sigma-Aldrich.

The N-protecting group can be subsequently removed, if desired, utilizing the procedures described above, and then reacted with a carboxylate represented by the formula: 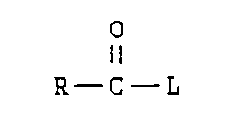 wherein R is as defined above and L is an appropriate leaving group such as a halide. Preferably, where R¹ is a side chain of a naturally occurring α-amino acid, R is a 2-quinoline group derived from N-hydroxysuccinimide-2-quinoline carboxylate, i.e., L is hydroxy succinimide. A solution of the free amine (or amine acetate salt) and about 1.0 equivalent of the carboxylate are mixed in an appropriate solvent system and optionally treated with up to five equivalents of a base such as, for example, N-methylmorpholine, at about room temperature. Appropriate solvent systems include tetrahydrofuran, methylene chloride or N,N-dimethylformamide, and the like, including mixtures thereof.

Contemplated equivalents of the general formula set forth above for the antiviral compounds and derivatives as well as the intermediates are compounds otherwise corresponding thereto and having the same general properties wherein one or more of the various R groups are simple variations of the substituents as defined therein, e.g., wherein R is a higher alkyl group than that indicated. In addition, where a substituent is designated as, or can be, a hydrogen, the exact chemical nature of a substituent which is other than hydrogen at that position, e.g., a hydrocarbyl radical or a halogen, hydroxy, amino and the like functional group, is not critical so long as it does not adversely affect the overall activity and/or synthesis procedure. The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

Examples 1-13 illustrate compounds wherein X is N rather than O or C(R¹⁷). However, as shown in Examples 14 and 15, the nitrogen can be replaced as shown in such Examples 14 and 15 by replacing the isocyanate R⁴NCO with an acid chloride or anhydride where X is C, or with a chloroformate or pyrocarbonate where X is 0, to produce the compounds of the present invention. Furthermore, as shown in Examples 16 and 17, such compounds are effective retroviral protease inhibitors.

All reagents were used as received without purification. All proton and carbon NMR spectra were obtained on either a Varian VXR-300 or VXR-400 nuclear magnetic resonance spectrometer.

### EXAMPLE 1

Additional exemplary compounds of the present invention are listed in Table 1. These compounds were prepared according to the following general procedures.

### General Procedure for the Synthesis of 1,3-Diamino 4-phenyl Butan-2-ol Derivatives.

A mixture of the amine R³NH₂ (20 equiv.) in dry isopropyl alcohol (20mL/mmol of epoxide to be converted) was heated to reflux and then treated with an N-Cbz amino epoxide of the formula: 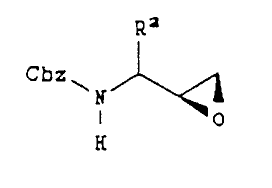 from a solids addition funnel over a 10-15 minute period. After the addition is complete the solution was maintained at reflux for an additional 15 minutes and the progress of the reaction monitored by TLC. In nearly all cases the reaction was found to be complete after this time period. The reaction mixture was then concentrated in vacuo to give an oil that was treated with n-hexane with rapid stirring whereupon the ring opened material precipitated from solution. Precipitation was generally complete within 1 hr and the product was then isolated by filtration on a Büchner funnel and then air dried. The product was further dried in vacuo. This method affords amino alcohols of sufficient purity for most purposes.

### General procedure for the Reaction of Amino Alcohols with Isocyanates: Preparation of Ureas

A solution from the amino alcohol in tetrahydrofuran (THF) was treated at room temperature with the appropriate isocyanate of formula R⁴NCO via syringe under nitrogen. After the reaction has stirred for ~5m the progress of the reaction was monitored by TLC. In nearly all cases the reaction was complete. The solvent was removed in vacuo and the product obtained was of sufficient purity for most purposes. The product may be further purified by dissolution in ethyl acetate and washing with 5% aqueous citric acid, water, and brine. The solvent is dried over anhydrous magnesium sulfate, filtered and concentrated in vacuo to give the pure urea.

### General Procedure for the Removal of the Protecting Groups by Hydrogenolysis with Palladium on Carbon

### A. Alcohol Solvent

The Cbz-protected peptide derivative was dissolved in methanol (ca.20mL/mmol) and 10% palladium on carbon catalyst is added under a nitrogen atmosphere. The reaction vessel is sealed and flushed 5 times with nitrogen and then 5 times with hydrogen. The pressure is maintained at 50 psig for 1-16 hours and then the hydrogen replaced with nitrogen and the solution filtered through a pad of celite to remove the catalyst. The solvent is removed in vacuo to give the free amino derivative of suitable purity to be taken directly on to the next step.

### B. Acetic Acid Solvent

The Cbz-protected peptide derivative was dissolved in glacial acetic acid (20mL/mmol) and 10% palladium on carbon catalyst is added under a nitrogen atmosphere. The reaction vessel is flushed 5 times with nitrogen and 5 times with hydrogen and then maintained at 40 psig for about 2h. The hydrogen was then replaced with nitrogen and the reaction mixture filtered through a pad of celite to remove the catalyst. The filtrate was concentrated and the resulting product taken up in anhydrous ether and evaporated to dryness 3 times. The final product, the acetate salt, was dried in vacuo and is of suitable purity for subsequent conversion.

### General Procedure for Removal of Boc-protecting Group with 4N Hydrochloric Acid in Dioxane

The Boc-protected amino acid or peptide is treated with a solution of 4N HCl in dioxane with stirring at room temperature. Generally the deprotection reaction is complete within 15 minutes, the progress of the reaction is monitored by thin layer chromatography (TLC). Upon completion, the excess dioxane and HCl are removed by evaporation in vacuo. The last traces of dioxane and HCl are best removed by evaporation again from anhydrous ether or acetone. The hydrochloride salt thus obtained is thoroughly dried in vacuo and is suitable for further reaction.

### EDC/HOBt Coupling of Cbz-Asparagine (General Procedure)

N-CBZ-(L-asparagine (1.10eq) and N-hydroxybenzotriazole (HOBt) (1.5eq) are dissolved in dry dimethylformamide (DMF) (2-5mL/mmol) and cooled in an ice bath. 1-(3-dimethylaminopropyl)-3-ethylcarbodiimide hydrochloride (EDC) (1.10eq) is added to the stirring solution and maintained at 0°C for 10 minutes. A solution of the amino component (free amine), 1.0eq in DMF (1-2mL/mmol), is added. [In the case of the amine hydrochloride or acetate salt, an equivalent of N-methylmorpholine is also added.] The reaction mixture is stirred at 0°C for 1 hour and then at room temperature for ~5-6 hours. The reaction mixture is then poured into a rapidly stirring solution of 60% saturated aqueous sodium bicarbonate (ca-50mL/mmol). An immediate white precipitate forms which is collected on a Buchner funnel and the solid washed thoroughly with saturated aqueous sodium bicarbonate, water, 5% aqueous citric acid solution and water. The product is thoroughly dried in vacuo and redissolved in DMF, filtered and reprecipitated by the addition to water. The precipitated product is isolated by filtration, washed again with water and dried in vacuo.

### General Procedure for Acylation with 2-Quinoline Carboxylic Acid N-Hydroxysuccinimide Ester

A solution of the free amine (or amine acetate salt) and 1.0 equivalent of N-hydroxysuccinimide 2-quinoline carboxylate in anhydrous dichloromethane was treated with 1.5 equivalents of N-methylmorpholine (NMM) at room temperature. The progress of the reaction was monitored by TLC and when the reaction was complete the reaction mixture was diluted with additional dichloromethane and the solution washed with saturated aqueous sodium bicarbonate, 5% aqueous citric acid, water and brine. The solution was dried over anhydrous magnesium sulfate, filtered and concentrated in vacuo. The product thus obtained was recrystallized from a mixture of acetone and hexane.

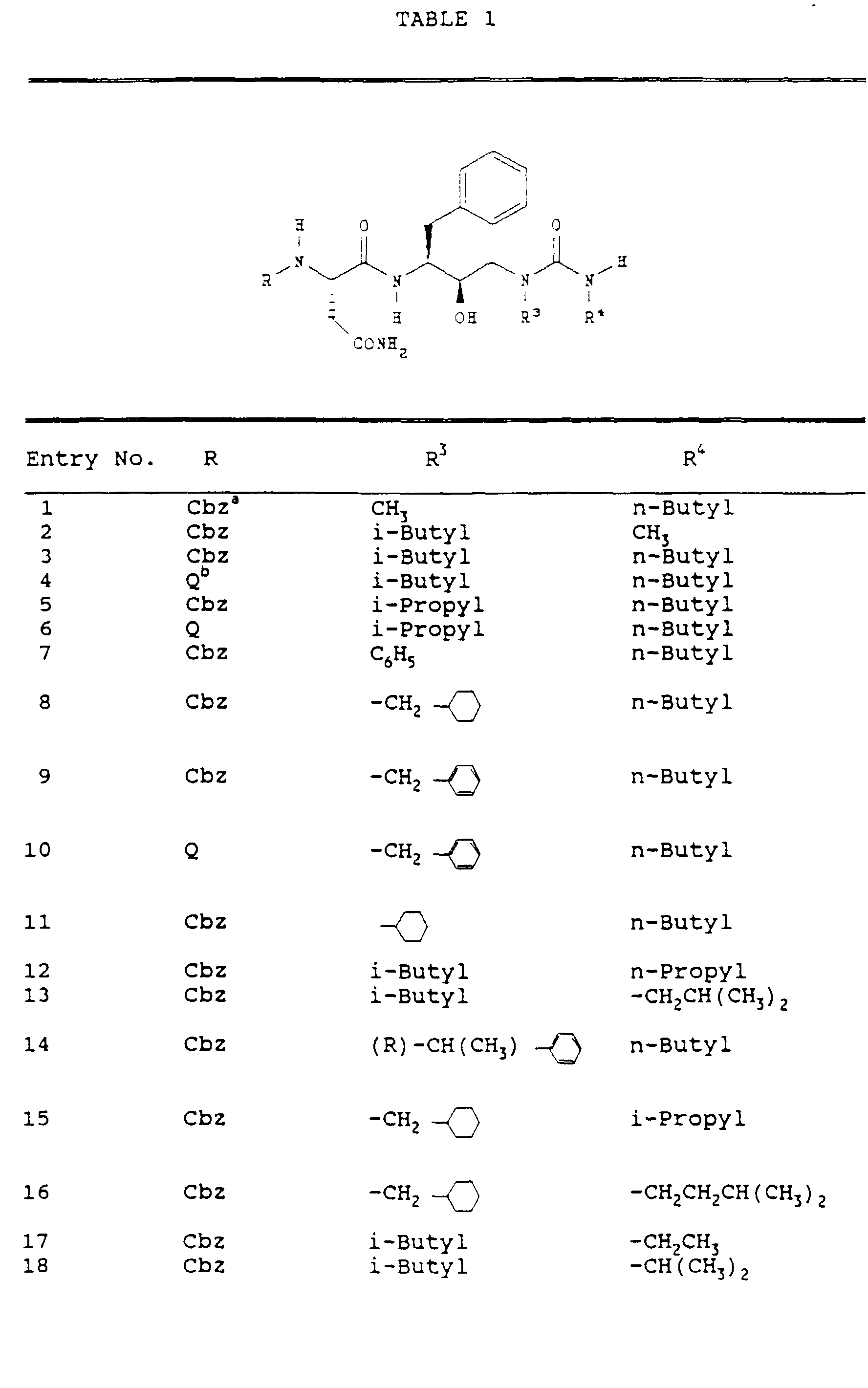

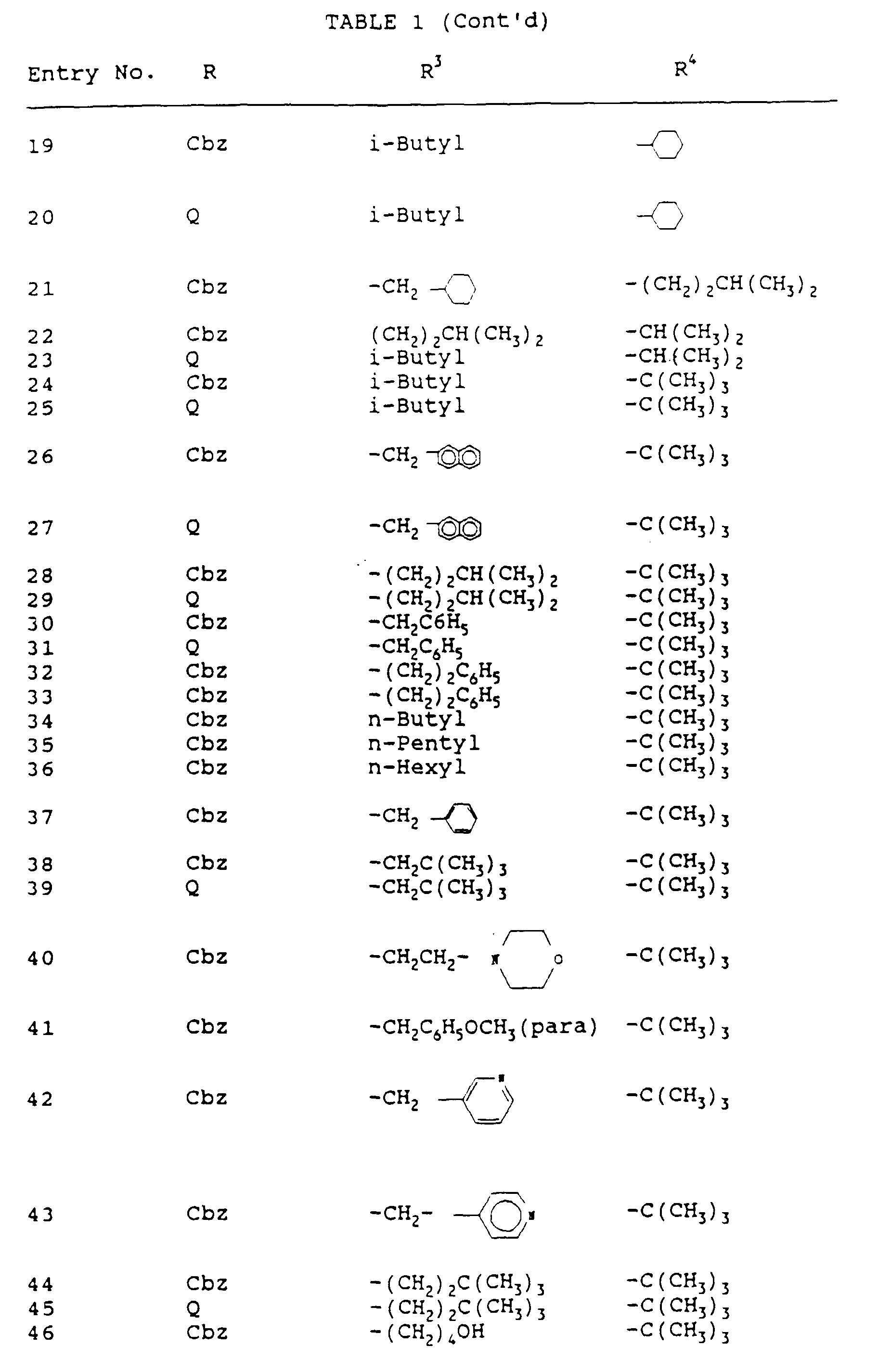

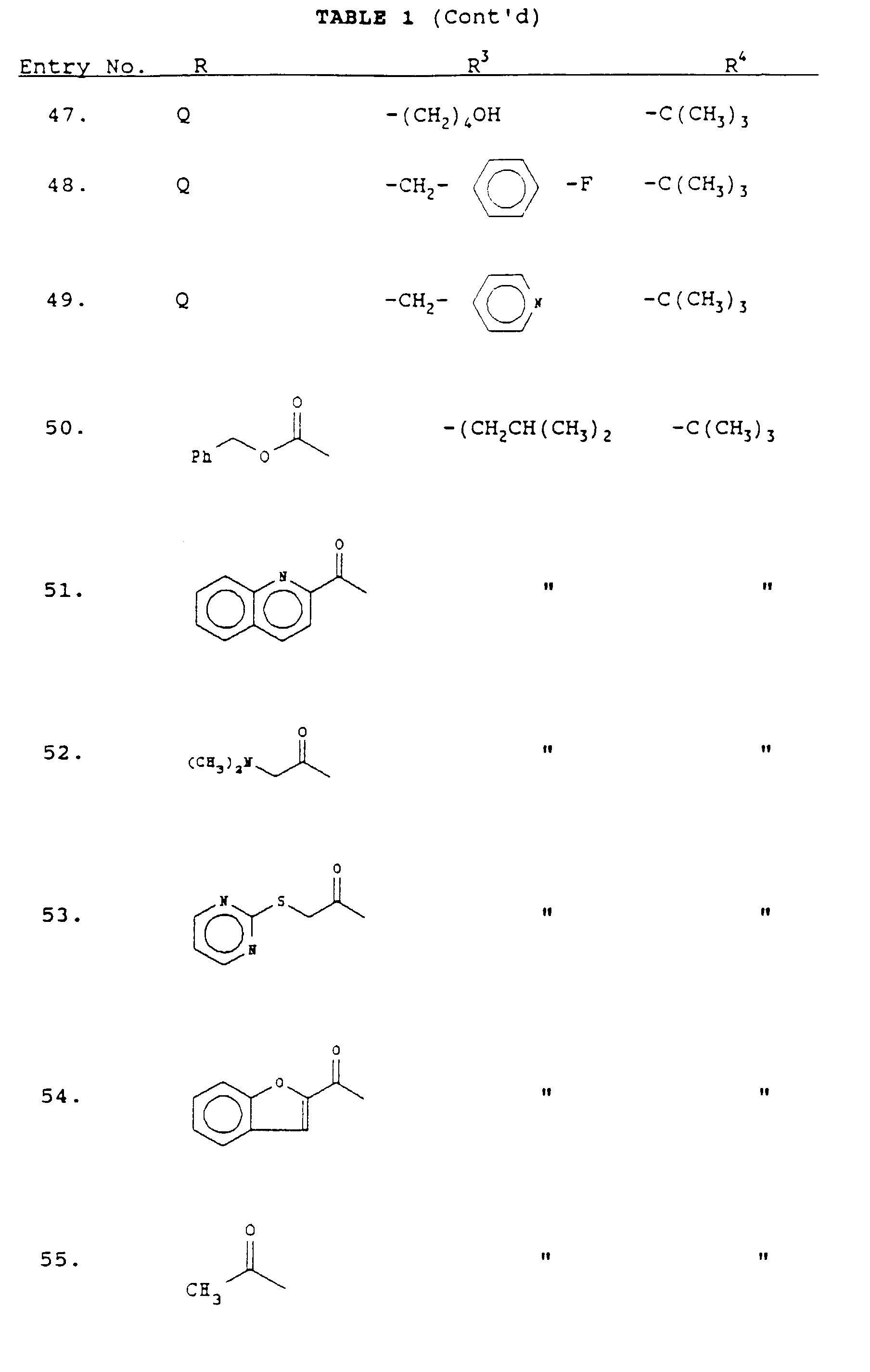

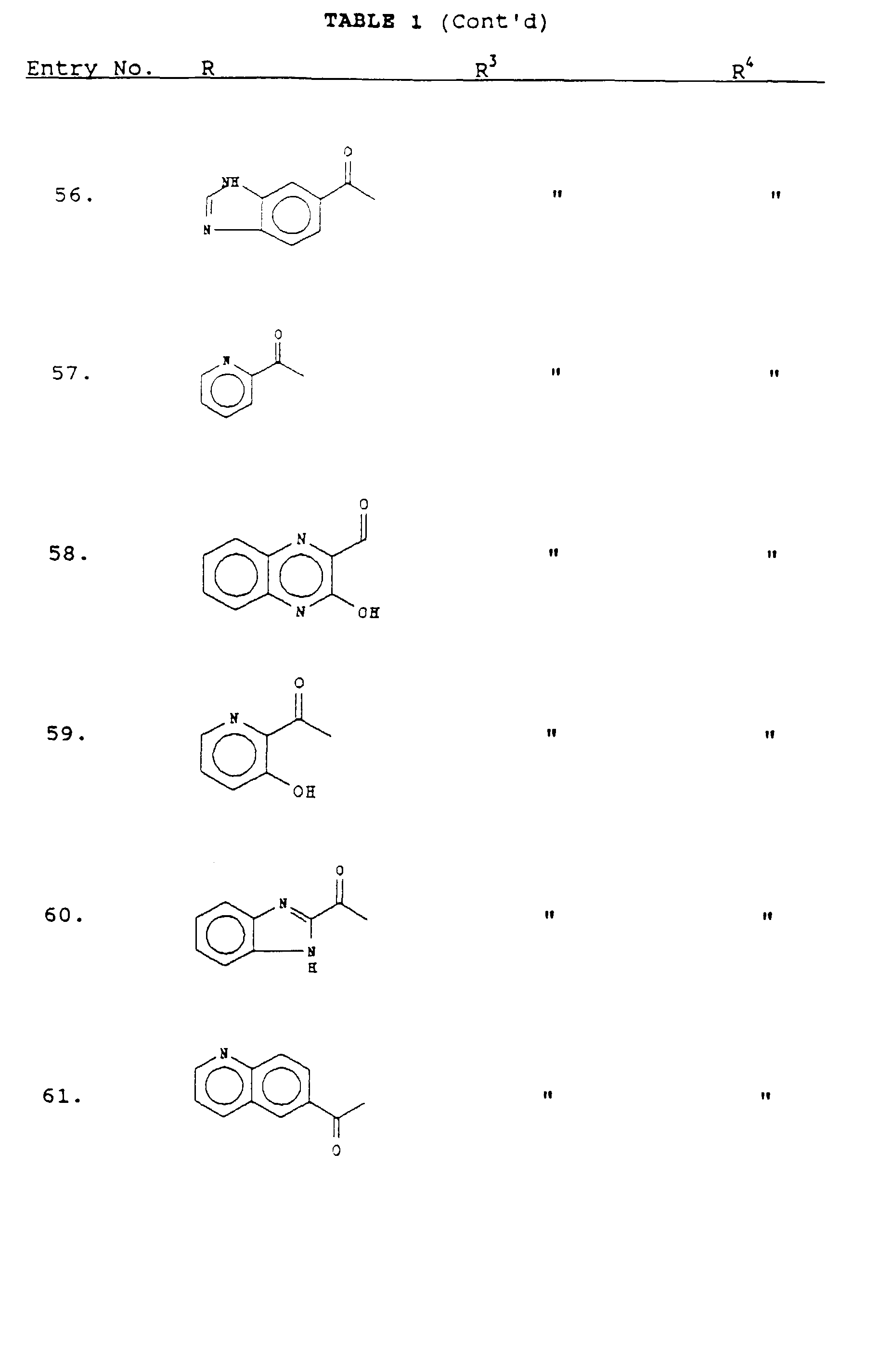

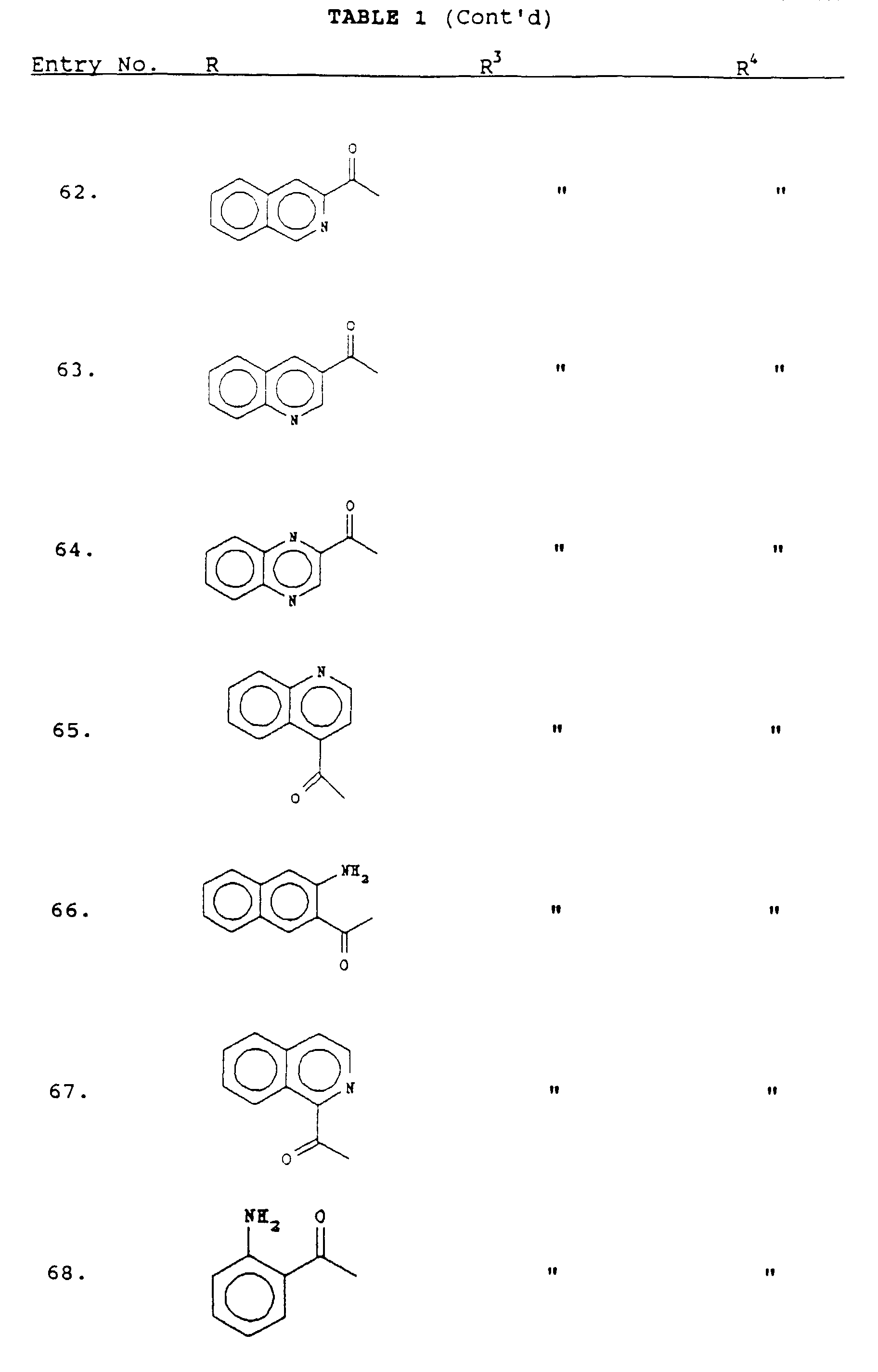

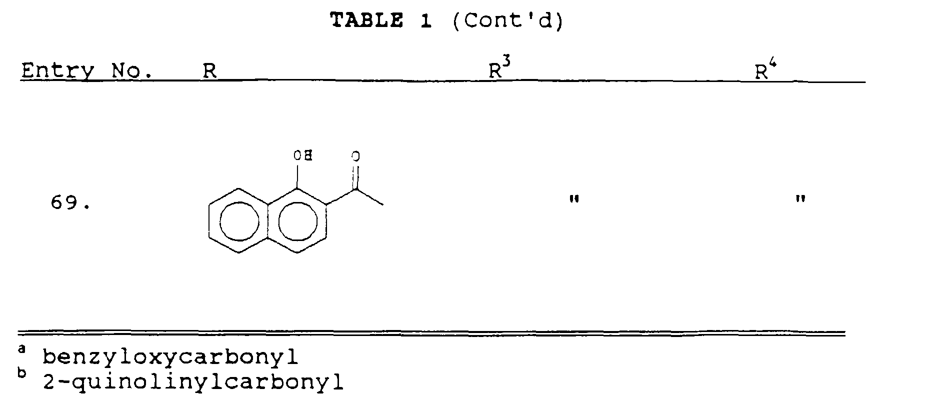

### EXAMPLE 2

Following the generalized procedures set forth in Example 1, the compounds set forth in Table 2 were prepared.

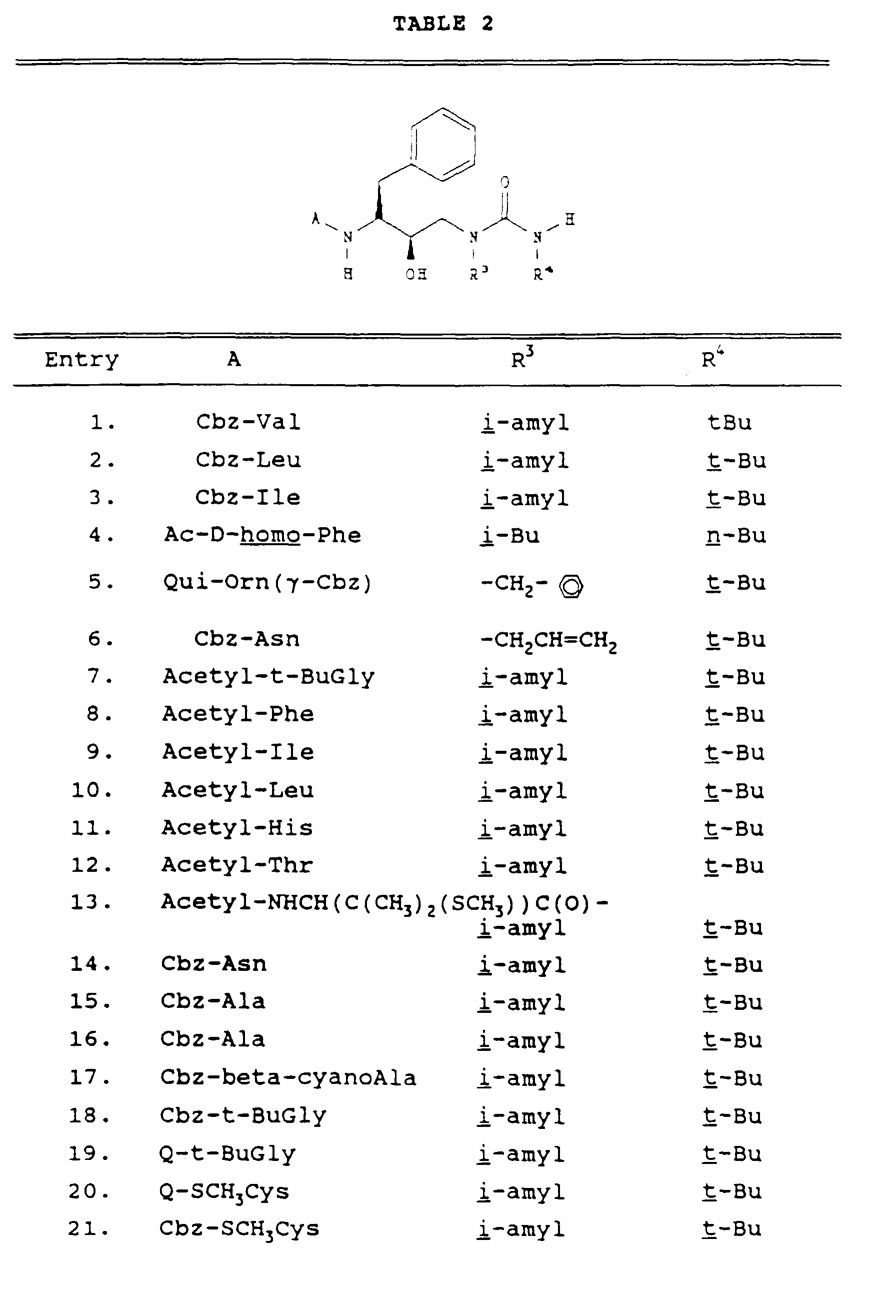

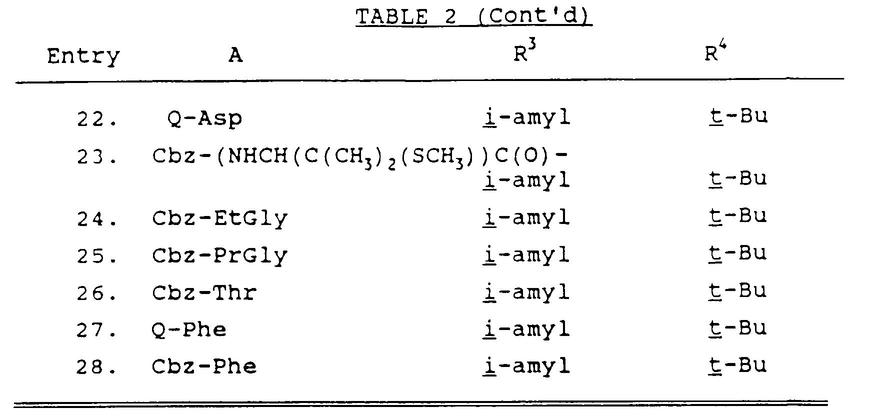

### EXAMPLE 3

Following the generalized procedure of Example 1, the compounds listed in Table 3 were prepared.

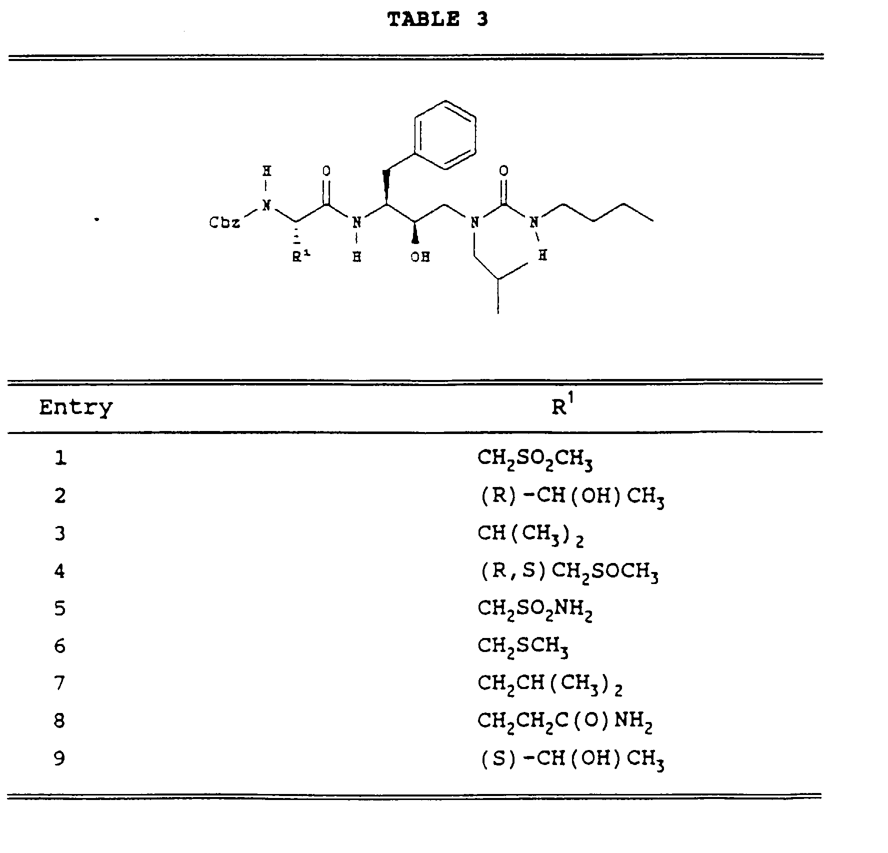

### EXAMPLE 4

Following the generalized procedures of Example 1, the compounds set forth in Table 4 were prepared.

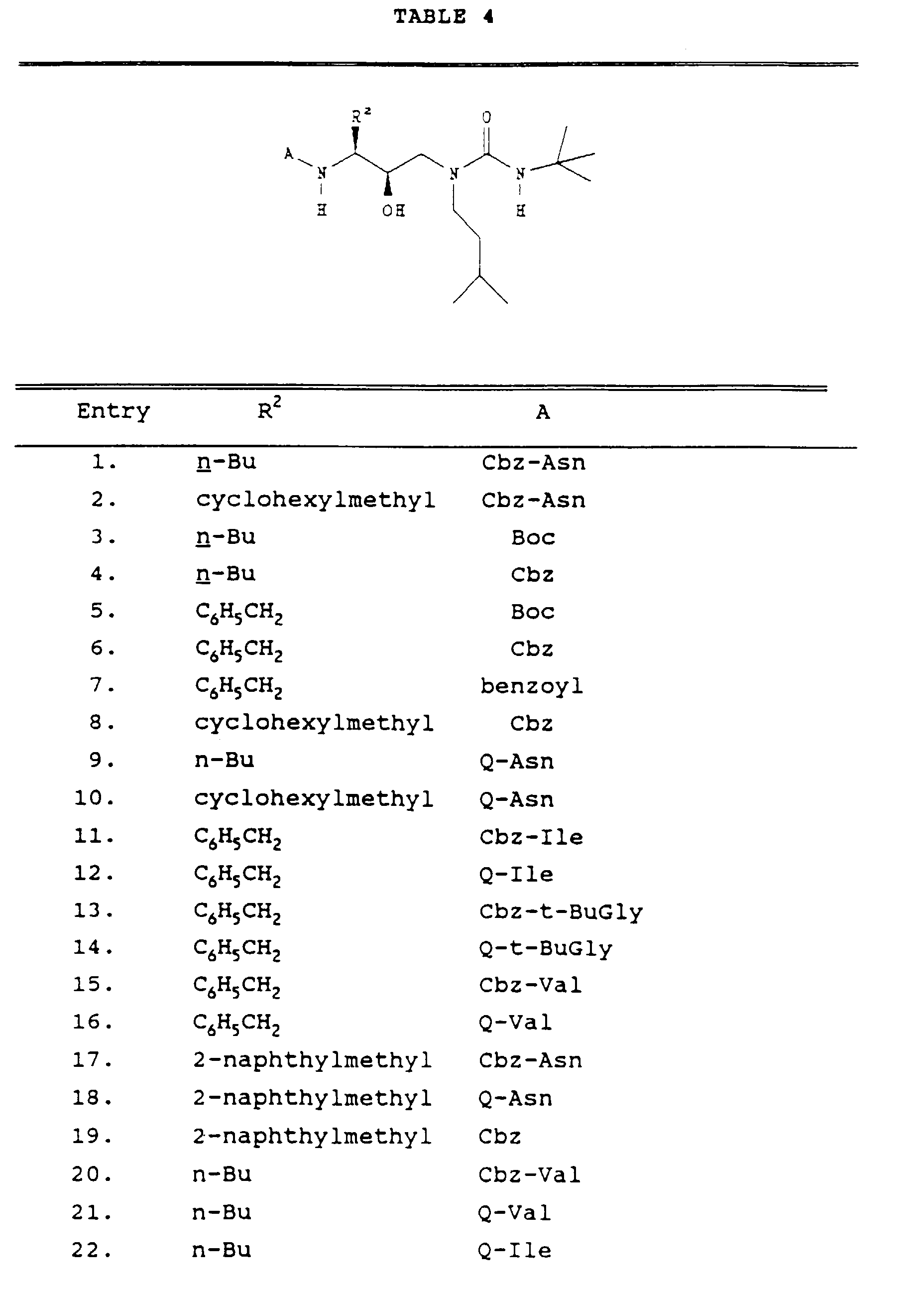

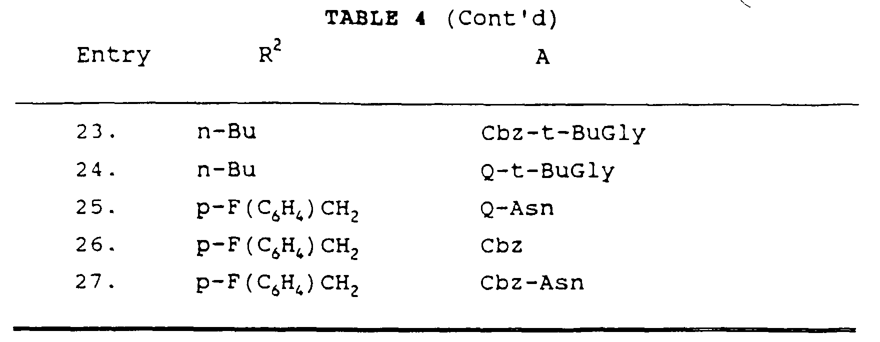

### EXAMPLE 5

The compounds listed in Table 5 were prepared according to the generalized procedures of Example 1.

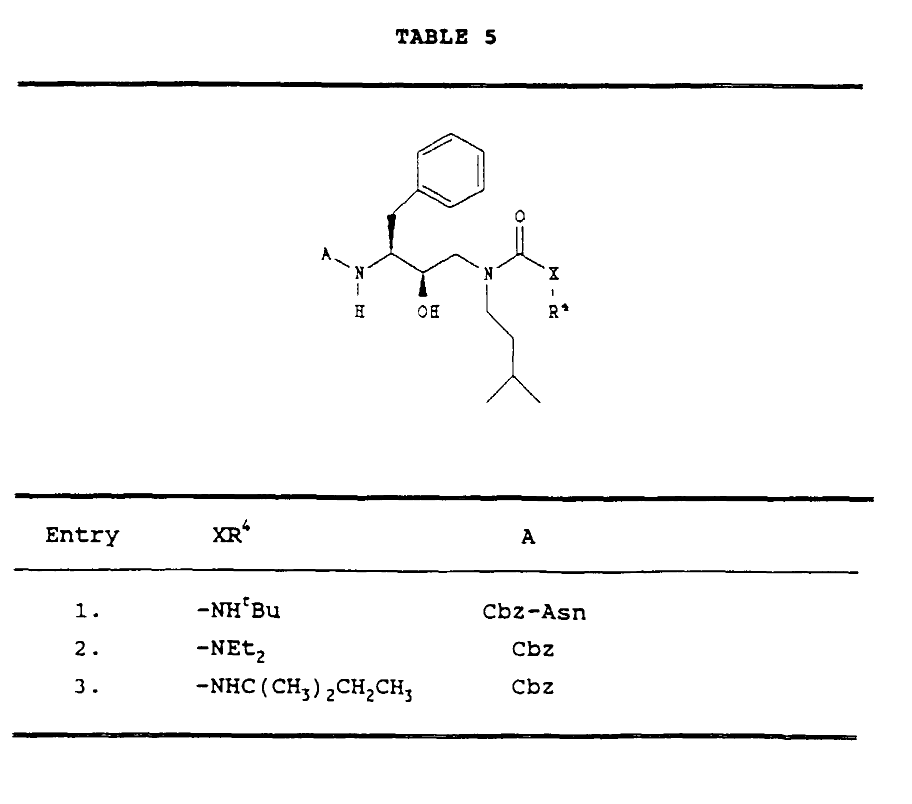

### EXAMPLE 6

The compounds of Table 6 were prepared according to the generalized procedures set forth in Example 1 except that instead of an isocyanate, an isothiocyanate equivalent was utilized.

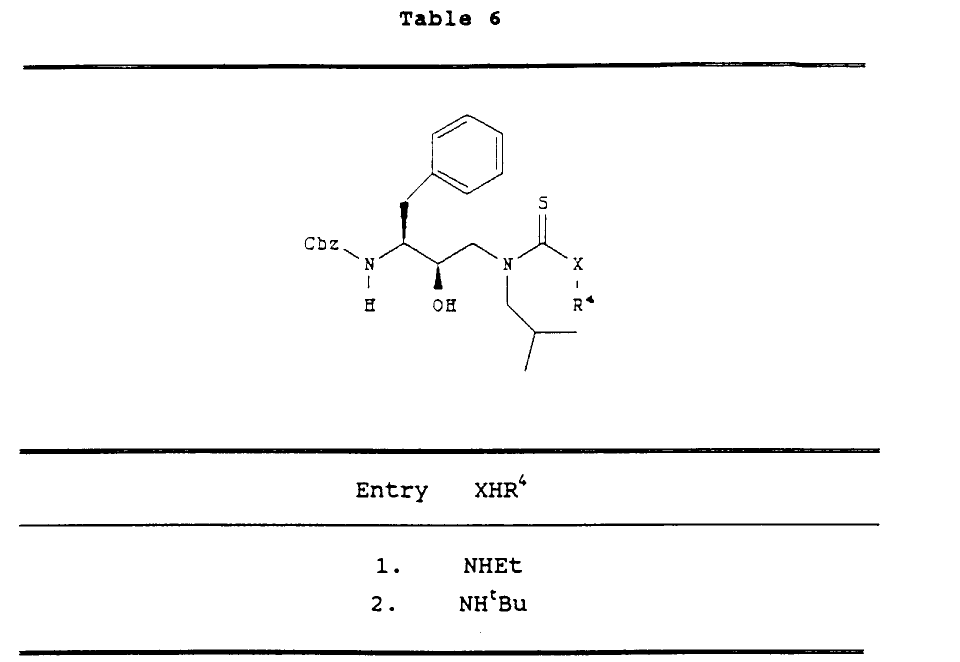

The Cbz group of the compounds shown in Examples 13 and 14 can be removed as described in Example 9 and the resulting compound can be coupled to a desired α- or β-amino acid or the like to produce compounds of the present invention.

### Example 7

### A. Preparation of 4(4-methoxybenzyl)itaconate

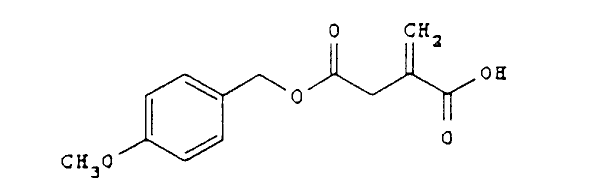

A 5 L three-necked round bottomed flask equipped with constant pressure addition funnel, reflux condenser, nitrogen inlet, and mechanical stirrer was charged with itaconic anhydride (660.8g, 5.88 mol) and toluene (2300 mL). The solution was warmed to reflux and treated with 4-methoxybenzyl alcohol (812.4g, 5.88 mol) dropwise over a 2.6h period. The solution was maintained at reflux for an additional 1.5h and then the contents were poured into three 2 L erlenmeyer flasks to crystallize. The solution was allowed to cool to room temperature whereupon the desired mono-ester crystallized. The product was isolated by filtration on a Buchner funnel and air dried to give 850.2g, 58% of material with mp 83-85°C, a second crop, 17% was isolated after cooling of the filtrate in an ice bath. ¹H NMR (CDCl₃) 300 MHz 7.32(d, J=8.7 Hz, 2H), 6.91(d, J=8.7 Hz, 2H), 6.49(s, 1H), 5.85(s, 1H), 5.12(s, 2H), 3.83(s, 3H), 3.40(s, 2H).

### B. Preparation of Methyl 4(4-methoxybenzyl) itaconate

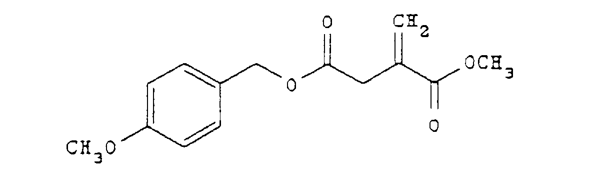

A 5 L three-necked round bottomed flask equipped with reflux condenser, nitrogen inlet, constant pressure addition funnel and mechanical stirrer was charged with 4(4-methoxybenzyl) itaconate (453.4g, 1.81 mol) and treated with 1,5-diazabicyclo[4.3.0]non-5-ene (275.6g, 1.81 mol), (DBU), dropwise so that the temperature did not rise above 15°C. To this stirring mixture was added a solution of methyl iodide (256.9g, 1.81 mol) in 250 mL of toluene from the dropping funnel over a 45m period. The solution was allowed to warm to room temperature and stirred for an additional 3.25h.

The precipitated DBU hydroiodide was removed by filtration, washed with toluene and the filtrate poured into a separatory funnel. The solution was washed with sat. aq. NaHCO₃ (2 X 500 mL), 0.2N HCl (1 X 500 mL), and brine (2 X 500 mL), dried over anhyd. MgSO₄, filtered, and the solvent removed *in vacuo.* This gave a clear colorless oil, 450.2g, 94% whose NMR was consistent with the assigned structure. ¹H NMR (CDCl₃) 300 MHz 7.30(d, J=8.7 Hz, 2H), 6.90(d, J=8.7 Hz, 2H), 6.34(s, 1H), 5.71(s, 1H), 5.09(s, 2H), 3.82(s, 3H), 3.73(s, 3H), 3.38(s, 2H). ¹³C NMR (CDl₃) 170.46, 166.47, 159.51, 133.55, 129.97, 128.45, 127.72, 113.77, 66.36, 55.12, 51.94, 37.64.

### C. Preparation of Methyl 4(4-methoxybenzyl) 2(R)-methylsuccinate

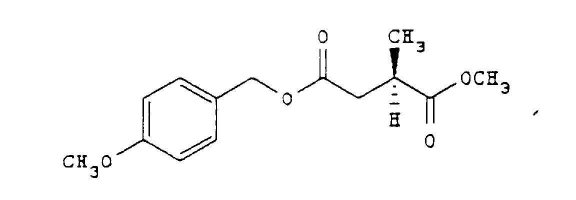

A 500 mL Fisher-Porter bottle was charged with methyl 4(4-methoxybenzyl) itaconate (71.1g, 0.269 mol), rhodium (R,R) DiPAMP catalyst (204mg, 0.269 mmol, 0.1 mol%) and degassed methanol (215 mL). The bottle was flushed 5 times with nitrogen and 5 times with hydrogen to a final pressure of 40 psig. The hydrogenation commenced immediately and after ca. 1h the uptake began to taper off, after 3h the hydrogen uptake ceased and the bottle was flushed with nitrogen, opened and the contents concentrated on a rotary evaporator to give a brown oil that was taken up in boiling *iso*-octane (ca. 200 mL, this was repeated twice), filtered through a pad of celite and the filtrate concentrated *in vacuo* to give 66.6g, 93% of a clear colorless oil, ¹H NMR (CDCl₃ 300 MHz 7.30(d, J=8.7 Hz, 2H), 6.91(d, J=8.7 Hz, 2H) , 5.08(s, 2H), 3.82(s, 3H), 3.67(s, 3H), 2.95(ddq, J=5.7, 7.5, 8.7 Hz, 1H), 2.79(dd, J=8.1, 16.5 Hz, 1H), 2.45(dd, J=5.7, 16.5 Hz, 1H), 1.23(d, J=7.5 Hz, 3H).

### D. Preparation of Methyl 2(R)-methylsuccinate

A 3 L three-necked round-bottomed flask equipped with a nitrogen inlet, mechanical stirrer, reflux condenser and constant pressure addition funnel was charged with methyl 4(4-methoxybenzyl) 2(R)-methylsuccinate (432.6g, 1.65 mol) and toluene (1200 mL). The stirrer was started and the solution treated with trifluoroacetic acid (600 mL) from the dropping funnel over 0.25h. The solution turned a deep purple color and the internal temperature rose to 45°C. After stirring for 2.25h the temperature was 27°C and the solution had acquired a pink color. The solution was concentrated on a rotary evaporator. The residue was diluted with water (2200 mL) and sat. aq. NaHCO₃ (1000 mL). Additional NaHCO₃ was added until the acid had been neutralized. The aqueous phase was extracted with ethyl acetate (2 X 1000 mL) to remove the by-products and the aqueous layer was acidified to pH=1.8 with conc. HCl. This solution was extracted with ethyl acetate (4 X 1000 mL), washed with brine, dried over anhyd. MgSO₄, filtered and concentrated on a rotary evaporator to give a colorless liquid 251g, >100% that was vacuum distilled through a short path apparatus cut 1: bath temperature 120°C @ >1mm, bp 25-29°C; cut 2: bath temperature 140°C @ 0.5mm, bp 95-108°C, 151g, [α]₀ @ 25°C=+1.38°C(c=15.475, MeOH), [α]_{D}=+8.48°C (neat); cut 3: bath temperature 140°C, bp 108°C, 36g, [α]_{D} @ 25°C=+1.49°C(c=15.00, MeOH), [α]_{D}=+8.98°C (neat). Cuts 2 and 3 were combined to give 189g, 78% of product, ¹H NMR (CDCl₃) 300 MHz 11.6(brs, 1H), 3.72(s, 3H), 2.92(ddq, J=5.7, 6.9, 8.0 Hz, 1H), 2.81(dd, J=8.0, 16.8 Hz, 1H), 2.47(dd, J=5.7, 16.8 Hz, 1H), 1.26(d, J=6.9 Hz, 3H).

### E. Preparation of Methyl Itaconate

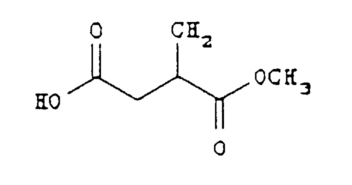

A 50 mL round bottomed flask equipped with reflux condenser, nitrogen inlet and magnetic stir bar was charged with methyl 4(4-methoxybenzyl) itaconate (4.00g, 16 mmol). The solution was kept at room temperature for 18 hours and then the volatiles were removed *in vacuo.* The residue was taken up in ethyl acetate and extracted three times with saturated aqueous sodium bicarbonate solution. The combined aqueous extract was acidified to pH=1 with aqueous potassium bisulfate and then extracted three times with ethyl acetate. The combined ethyl acetate solution was washed with saturated aqueous sodium chloride, dried over anhydrous magnesium sulfate, filtered, and concentrated *in vacuo.* The residue was then vacuum distilled to give 1.23g, 75% of pure product, bp 85-87 @ 0.1 mm. ¹H NMR (CDCl₃) 300 MHz 6.34(s, 1H), 5.73(s, 2H), 3.76(s, 3H), 3.38(s, 2H). ¹³C NMR (CDCl₃) 177.03, 166.65, 129.220, 132.99, 52.27, 37.46.

### F. Curtius Rearrangement of Methyl 2(R)-methylsuccinate: Preparation of Methyl N-Moz-α-methyl β-alanine.

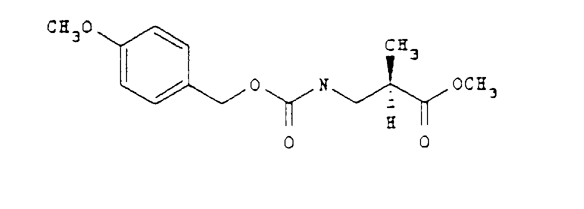

A 5L four"necked round bottomed flask equipped with a nitrogen inlet, reflux condenser, mechanical stirrer, constant pressure addition funnel, and thermometer adapter was charged with methyl 2(R)-methylsuccinate (184.1g, 1.26 mol), triethylamine (165.6g, 218 mL, 1.64 mol, 1.3 equivalents), and toluene (1063 mL). The solution was warmed to 85°C and then treated dropwise with a solution of diphenylphosphoryl azide (346.8g, 1.26 mol) over a period of 1.2h. The solution was maintained at that temperature for an additional 1.0h and then the mixture was treated with 4-methoxybenzyl alcohol (174.1g, 1.26 mol) over a 0.33h period from the dropping funnel. The solution was stirred at 88°C for an additional 2.25h and then cooled to room temperature. The contents of the flask were poured into a separatory funnel and washed with sat. aq. NaHCO₃ (2 X 500 mL), 0.2N HCl (2 X 500 mL), brine (1 X 500 mL), dried over anhyd. MgSO₄, filtered, and concentrated *in vacuo* to give 302.3g, 85% of the desired product as a slightly brown oil. ₁H NMR (CDCl₃) 300 MHz 7.32(d, J=8.4 Hz, 2H), 6.91(d, J=8.4 Hz, 2H), 5.2(brm, 1H), 5.05(s, 2H), 3.83(s, 3H), 3.70(s, 3H), 3.35(m, 2H), 2.70(m, 2H), 1.20(d, J=7.2 Hz, 3H).

### G. Hydrolysis of Methyl N-Moz-α-methyl β-alanine: Preparation of α-methyl β-alanine Hydrochloride

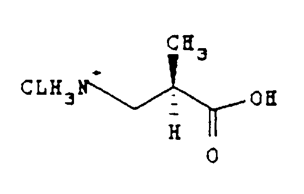

A 5 L three-necked round bottomed flask equipped with a reflux condenser, nitrogen inlet and mechanical stirrer was charged with methyl N-Moz-α-methyl β-alanine (218.6g, 0.78 mol), glacial acetic acid (975 mL) and 12N hydrochloric acid (1960 mL). The solution was then heated to reflux for 3h. After the solution had cooled to room temperature (ca. 1h) the aqueous phase was decanted from organic residue (polymer) and the aqueous phase concentrated on a rotary evaporator. Upon addition of acetone to the concentrated residue a slightly yellow solid formed that was slurried with acetone and the white solid was isolated by filtration on a Buchner funnel. The last traces of acetone were removed by evacuation to give 97.7g, 90% of pure product, mp 128.5-130.5°C [α]₀ @ 25°C=9.0°C (c=2.535, Methanol). ¹H NMR (D₂O) 300 MHz 3.29(dd, J=8.6, 13.0 Hz, 1H), 3.16(dd, J=5.0, 13.0m Hz, 1H), 2.94(ddq, J=7.2, 5.0, 8.6 Hz, 1H), 1.30(d,J=7.2 Hz, 3H); ¹³C NMR (D₂O) 180.84, 44.56, 40.27, 17.49.

### H. Preparation of N-Boc α-Methyl 8-Alanine

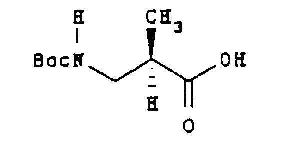

A solution of α-methyl β-alanine hydrochloride (97.7g, 0.70 mol) in water (1050 mL) and dioxane (1050 mL) the pH was adjusted to 8.9 with 2.9N NaOH solution. This stirring solution was then treated with di-*tert*-butyl pyrocarbonate (183.3g, 0.84 mol, 1.2 equivalents) all at once. The pH of the solution was maintained between 8.7 and 9.0 by the periodic addition of 2.5N NaOH solution. After 2.5h the pH had stabilized and the reaction was judged to be complete. The solution was concentrated on a rotary evaporator (the temperature was maintained at <40°C). The excess di-*tert*-butyl pyrocarbonate was removed by extraction with dichloromethane and then the aqueous solution was acidified with cold IN HCl and immediately extracted with ethyl acetate (4 X 1000 mL). The combined ethyl acetate extract was washed with brine, dried over anhyd. MgSO₄, filtered and concentrated on a rotary evaporator to give a thick oil 127.3g, 90% crude yield that was stirred with n-hexane whereupon crystals of pure product formed, 95.65g, 67%, mp 76-78°C, [α]_{D} @ 25°C=-11.8°C (c=2.4, EtOH). A second crop was obtained by concentration of the filtrate and dilution with hexane, 15.4g, for a combined yield of 111.05g, 78%. ¹H NMR (acetone D₆) 300 MHz 11.7 (brs, 1H), 6.05 (brs 1H), 3.35 (m, 1H), 3.22 (m, 1H), 2.50 (m, 1H), 1.45(s, 9H), 1.19 (d, J=7.3 Hz, 3H); ¹³C NMR (acetone D₆) 177.01, 79.28, 44.44, 40.92, 29.08, 15.50. Elemental analysis calc'd. for C₉H₁₇NO₄: C, 53.19, H, 8.42; N, 6.89. Found: C, 53.36; H, 8.46; N, 6.99.

### I. Preparation of N-4-Methoxybenzyloxycarbonyl α-Methyl β-Alanine

A solution of N-4-methoxybenzyloxycarbonyl α-methyl β-alanine methyl ester (2.81g, 10.0 mmol) in 30 mL of 25% aqueous methanol was treated with lithium hydroxide (1.3 equivalents) at room temperature for a period of 2h. The solution was concentrated *in vacuo* and the residue taken up in a mixture of water and ether and the phases separated and the organic phase discarded. The aqueous phase was acidified with aqueous potassium hydrogen sulfate to pH=1.5 and then extracted three times with ether. The combined ethereal phase was washed with saturated aqueous sodium chloride solution, dried over anhydrous magnesium sulfate, filtered and concentrated in *vacuo* to give 2.60 g, 97% of N-4-Methoxybenzyloxycarbonyl α-methyl β-alanine (N-Moz-AMBA) which was purified by recrystallization from a mixture of ethyl acetate and hexane to give 2.44g, 91% of pure product, mp 96-97°C, MH+=268. ¹H NMR (D₆-acetone/300 MHz) 1.16 (3H, d, J=7.2Hz), 2.70 (1H, m), 3.31 (2H, m), 3.31 (3H, s), 4.99 (2H, s), 6.92 (2H, 4, J=8.7 Hz), 7.13 (2H, d, J=8.7 Hz).

### J. Preparation of Propanamide, 3-(4-methoxybenzyloxycarbonyl)-N_[3-[[[(1,1-dimethylethyl)amine]carbonyl](3-methylbutyl)amino]-2-hydroxy-1-(phenylmethyl)propyl]-2-methyl-[IS-[IR*(S*), 25*]]-

N-Moz-AMBA (468mg, 1.75mmol) was dissolved in 5mL of DMF, HOBT (355mg, 2.6mmol) was added and the solution was cooled to 0°C. The solution was treated with (336mg, 1.75mmol) EDC for 15 minutes. To this was added (612mg, 1.75mmol) of [2R,3S 3-amino-1-isoamyl-1-(t-butylcarbonyl)amino 4-phenyl-2-butanol in 10mL of DMF and the reaction stirred for 16 hours at room temperature. The DMF was concentrated to 5mL and the product was precipitated by addition to 60% saturated aqueous NaHCO₃. The solid was taken up in ethyl acetate and washed with KHSO₄, NaHCO₃, NaCl(saturated), dried over MgSO₄ and concentrated to yield 680mg of crude product which was crystallized from CH₂Cl₂, Et₂O, hexane, to yield 300mg of pure product.

### Example 8

The compounds of Table 8 were prepared according to the procedure listed below and that utilized in Example 7.

### Propaneamide, 3-[(1,1-dimethylethyl)butoxycarbonyl]amino-N-[3-[[[(1,1--dimethylethyl)amino]carbonyl](3-methylbutyl)aminol-2-hydroxy-1-(phenylmethyl)propyl]-2-methyl-,[1S-[1R*(S*),2S*]-

### Part A.

A solution of N-t-butyloxycarbonyl-2-(R)-methyl-3-aminopropionic acid (372 mg, 1.83 mmol) and N-hydroxybenzotriazole (371 mg, 2.75 mmol) in 5 mL of dimethylformamide was cooled to 0 degrees C. To this was added EDC (351 mg, 1.83 mmol) and the solution was stirred for 15 minutes. To this chilled solution was added a solution of
3-[[[(1,1-dimethylethyl)amino]carbonyl](3-methylbutyrl)amino]-2(R)-hydroxy-1(S)(phenylmethyl)propylamine in 5 mL of dimethylformamide and stirred for 15 hours. The dimethylformamide was removed and replaced with 50 mL of ethyl acetate, and the organic phase was extracted with 5% potassium hydrogen sulfate, saturated sodium bicarbonate and brine. The ethyl acetate layer was dried over magnesium sulfate, filtered and concentrated to yield 613 mg of product after recrystallization from ethyl acetate , hexanes. (63 % yield). M+Li 541

### Part B.

### Preparation of Propaneamide,_3-amino-N-[3-[[[(1,1-dimethylethyl)amino] carbonyl]- (3-methylbutyl)amino]-2-hydroxy-1-(phenylmethyl)propyl]-2-methyl-,[1S-[1R*(S*), 2S*]-hydrochloride

The product from part A. (577 mg, 1.08 mmol) was dissolved in 40 mL of 4N HCl in dioxane and the solution stirred for 2 hours, and concentrated to yield the hydrochloride salt in quantitative yield.

### Part C.

### Preparation of Propaneamide, 3-(2-methylpropanoylamino)-N-[3-[[[(1,1-dimethylethyl)-amino]carbonyl](3-methylbutyl)amino]-2-hydroxy-1-(phenylmethyl)propyl]-2-methyl-,[1S-[1R*(S*),2S*]-

The product from part B. (236 mg, 0.5 mmol) was dissolved in anhydrous tetrahydrofuran and to this was added N-methylmorpholine (160 mg, 1.5 mmol) upon which time a precipitate formed. To this suspension was added isobutyryl chloride ( 53.5 mg, 0.5 mmol) and the suspension stirred for 15 hours. The suspension was diluted with ethyl acetate and washed with 5% potassium hydrogen sulfate, saturated sodium bicarbonate and brine. the organic layer was dried over magnesium sulfate, filtered and concentrated to yield 195 mg of crude product which was chromatographed on silica gel with 5% methanol methylene chloride to yield 121.5 mg ( 50 % yield) of pure product. M+Li 511

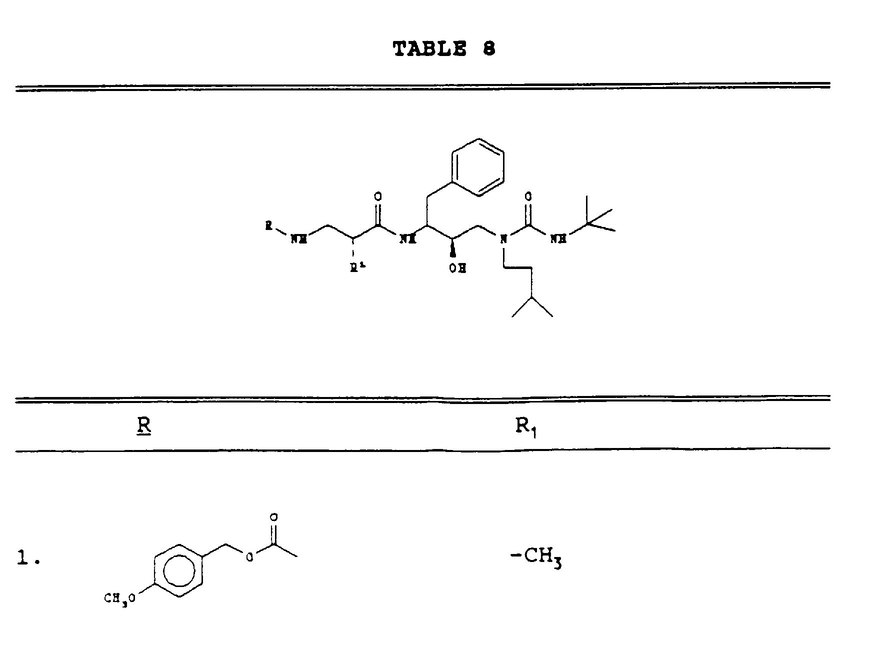

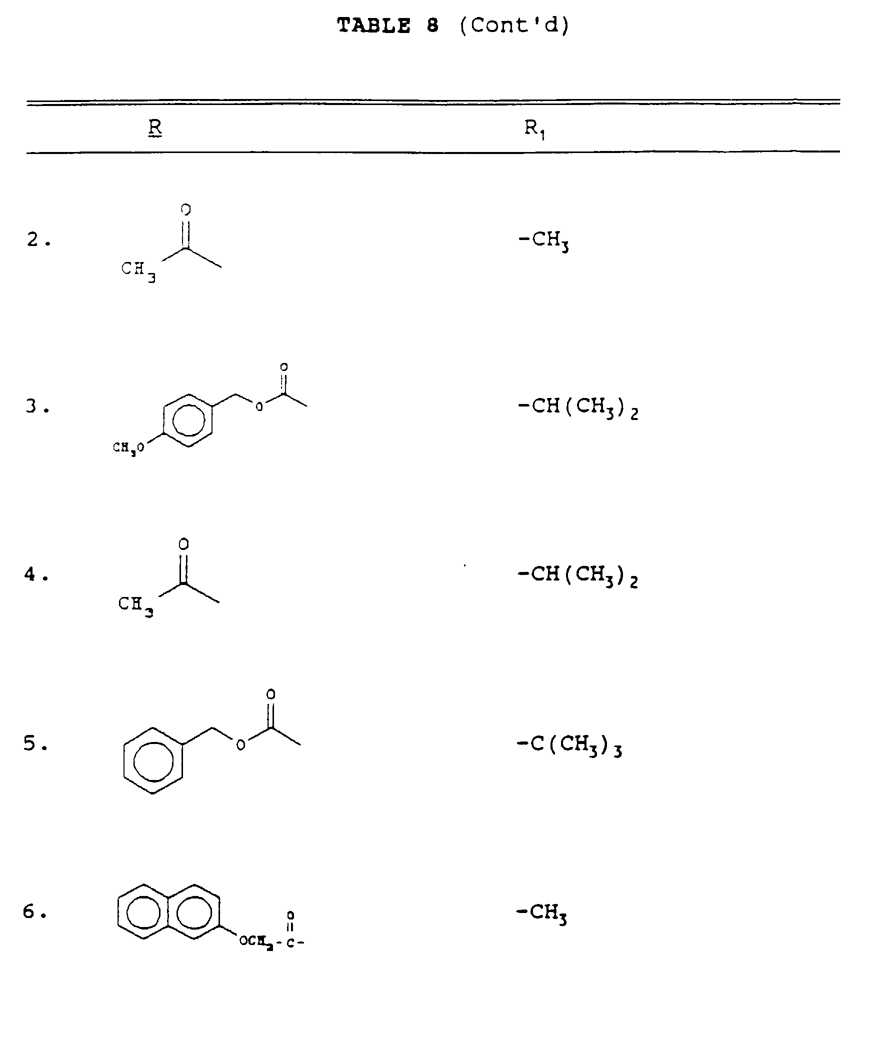

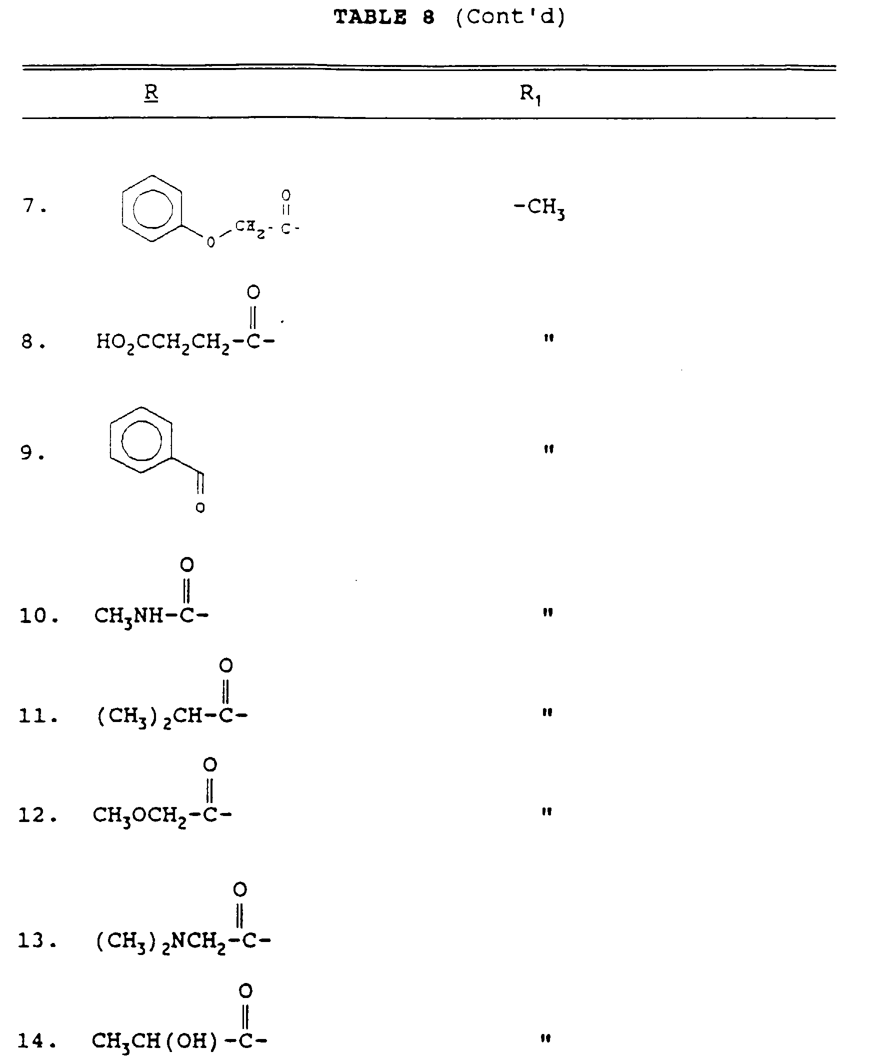

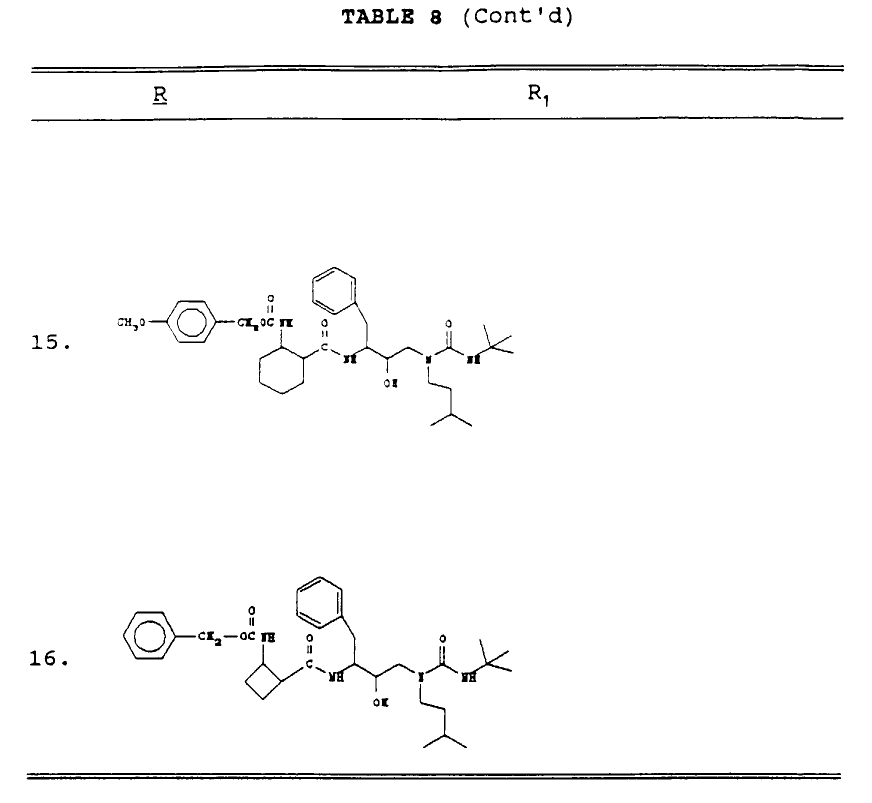

### Example 9

Following generally the procedure set forth in Example 7, the compounds shown in Table 9 were prepared.

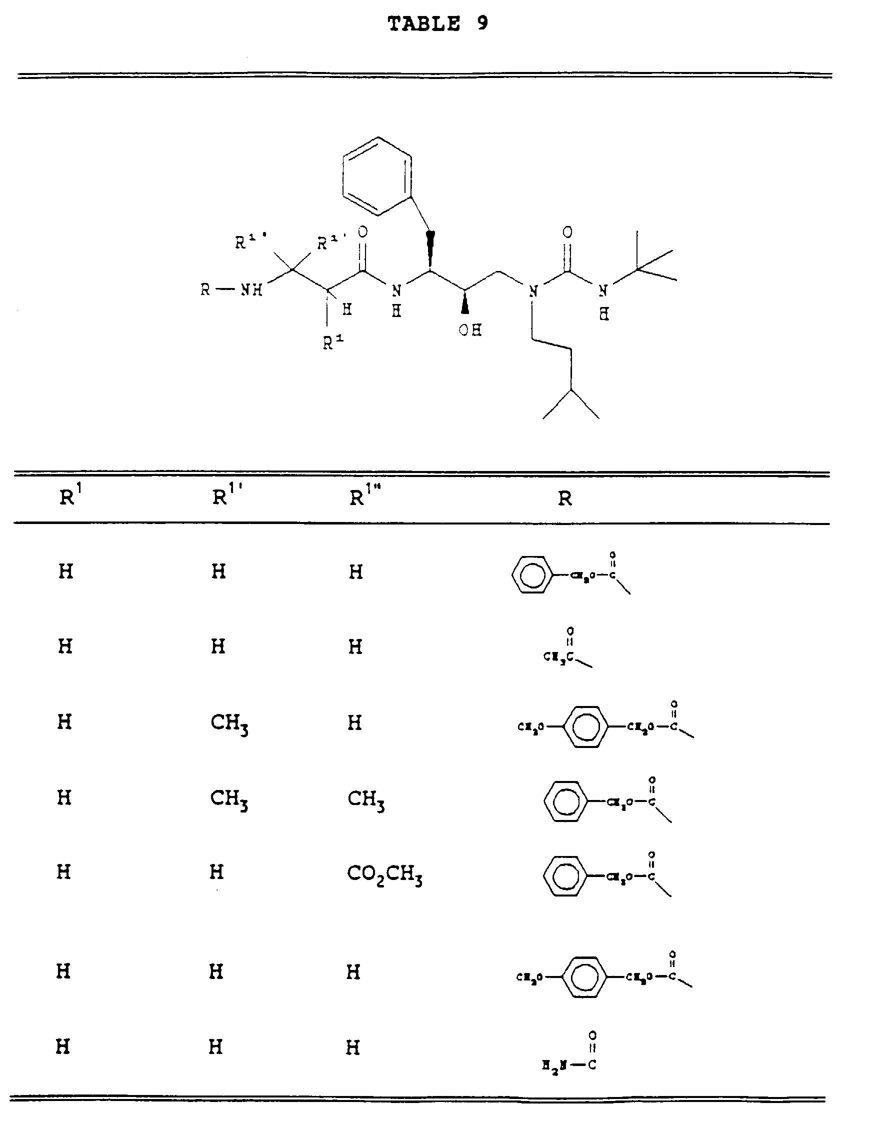

### Example 10

The procedure set forth below was generally utilized to prepare the compounds shown in Table 9

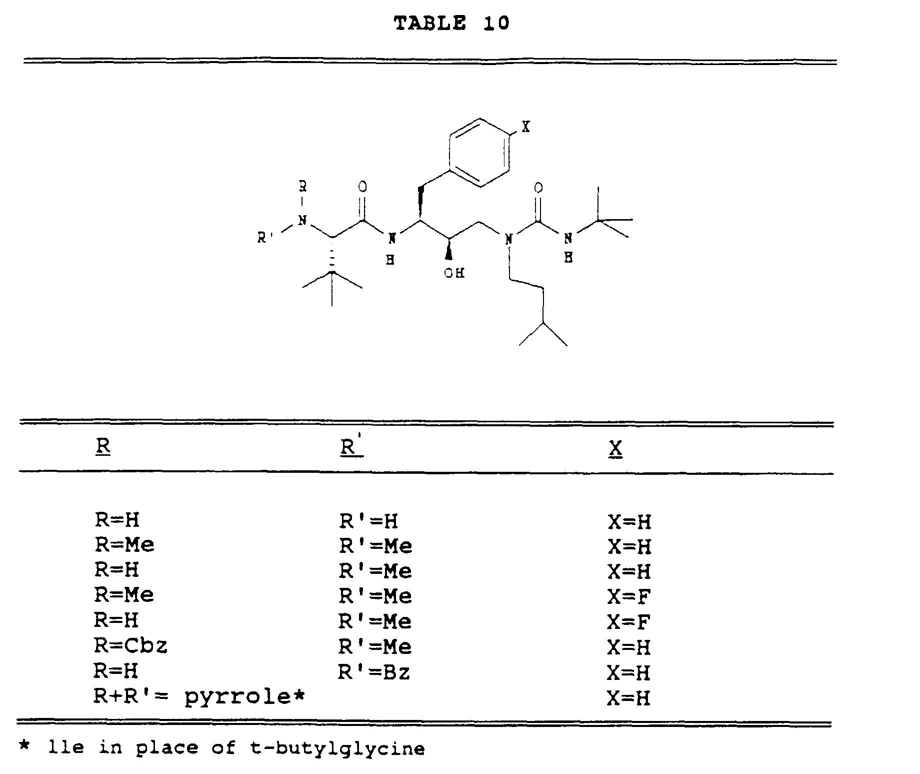

### Example 11

### 3-[[(1,1-dimethylethyl)amino]carbonyl](3-methylbutyl)amino-2(R)-hydroxy-1(S)-(phenylmethyl)propyl amine

This example illustrates preparation of compounds of Formula II wherein R¹ is an alkyl group other than an alkyl group of a naturally occurring amino acid side chain.

### Part A:

3-[[(1,1-dimethylethyl)amino]carbonyl](3-methylbutyl)amino-2(R)-hydroxy-1(S)-[N-(benzyloxycarbonyl)(phenylmethyl()propyl amine] (4.7 gm, 9.7 mmol) was combined with 10% Pd on carbon (200 mg) and conc. HCl (3 mL) in ethanol (35 mL) and hydrogenated at 50 psi of hydrogen for 2.5 h. The reaction mixture was filtered through diatomaceous earth and concentrated on a rotary evaporator to a yellow hygroscopic solid; 3.7 gm, 100%.

### Part B:

### Butaneamide, 2-[(phenylmethyloxycarbonyl)amino]-N-[3-[[[(1,1-dimethylethyl)amino]carbonyl](3-methylbutyl)amino]-2-hydroxy-1-(phenylmethyl)propyl-3,3-dimethyl-[1S-[1R*(R*),25*]]-

N-Cbz-L-tert-leucine (172 mg, 0.65 mmol) and N-hydroxybenzotriazole (100 mg, 0.65 mmol) in DMF (3 mL) was cooled to 0 C and EDC (115 mg, 0.60 mmol) added. After 45 min the amine from Part A (193 mg, 0.50 mmol) and N-methylmorpholine (60 uL, 0.55 mmol) were added. The reaction was stirred at ambient temperature for 18 h and poured into a solution of 50% saturated NaHCO₃ (25 mL). The solid was collected by suction filtration, washed with water and dried in-vacuo. The solid was chromatographed on SiO₂ using 2% MeOH in CH₂Cl₂. The appropriate fractions were pooled and concentrated to afford a white solid; 220 mg, MH* 597, TLC (SiO₂ 2%MeOH/CH₂Cl₂) R_{f} = .2 . CHN requires: C, 68.42, H, 8.78, N, 9.39; found: C, 68.03, H, 8.83, N, 9.33.

### Part C:

### Butaneamide, 2-amino-N-[3-[[[(1,1-dimethylethyl)amino]carbonyl](3-methylbutyl)amino]-2-hydroxy-1-(phenylmethyl)propyl-3,3-dimethyl-, [1S-[1R*(R*), 2S*]-

The product from Part B (570 mg, 0.95 mmol) and 4% Pd on carbon (150 mg) in ethanol (30 mL) was hydrogenated at 5 psi for 2.75 h. The reaction mixture was filtered through diatomaceous earth and concentrated on a rotary evaporator to an oil; 438 mg, 100%.

### Part D:

### Butaneamide, 2-(acetylamino)-N-[3-[[[(1,1-dimethylethyl)amino]carbonyl](3-methylbutyl)amino] -2-hydroxy-1-(phenylmethyl)propyl-3,3-dimethyl-, [1S-[1R*(R*), 2S*]-

The product from Part C (206 mg, 0.41 mmol) and N-methylmorpholine (45 uL, 0.41 mmol) were dissolved in CH₂Cl₂ (2.5 mL) and cooled to 0 C. Acetic anhydride (39 uL, 0.41 mmol) was then added and the reaction stirred 30 min at 0 C, then allowed to warm to ambient temperature and stir for 30 min. The solvent was removed on a rotary evaporator and the residue dissolved in ethanol (2 mL). The ethanolic solution was slowly poured into 50 % saturated NaHCO₃ (20 mL) and stirred vigorously. The solid was collected by suction filtration and washed with water, 5% citric acid, and again with water; 157 mg, 75%. CHN / 1.5 H₂O requires: C 63.24, H, 9.67, N, 10.54; found:
C, 63.40, H, 9.41, N, 10.39.

Butaneamide, 2-amino-N-[3-[[[(1,1-dimethylethyl)amino]carbonyl](3-methylbutyl)amino]-2-hydroxy-1-(phenylmethyl)propyl-3,3-dimethyl-, [1S-[1R*(R*), 2S*]- was also capped with the acyl groups shown in Table 12.

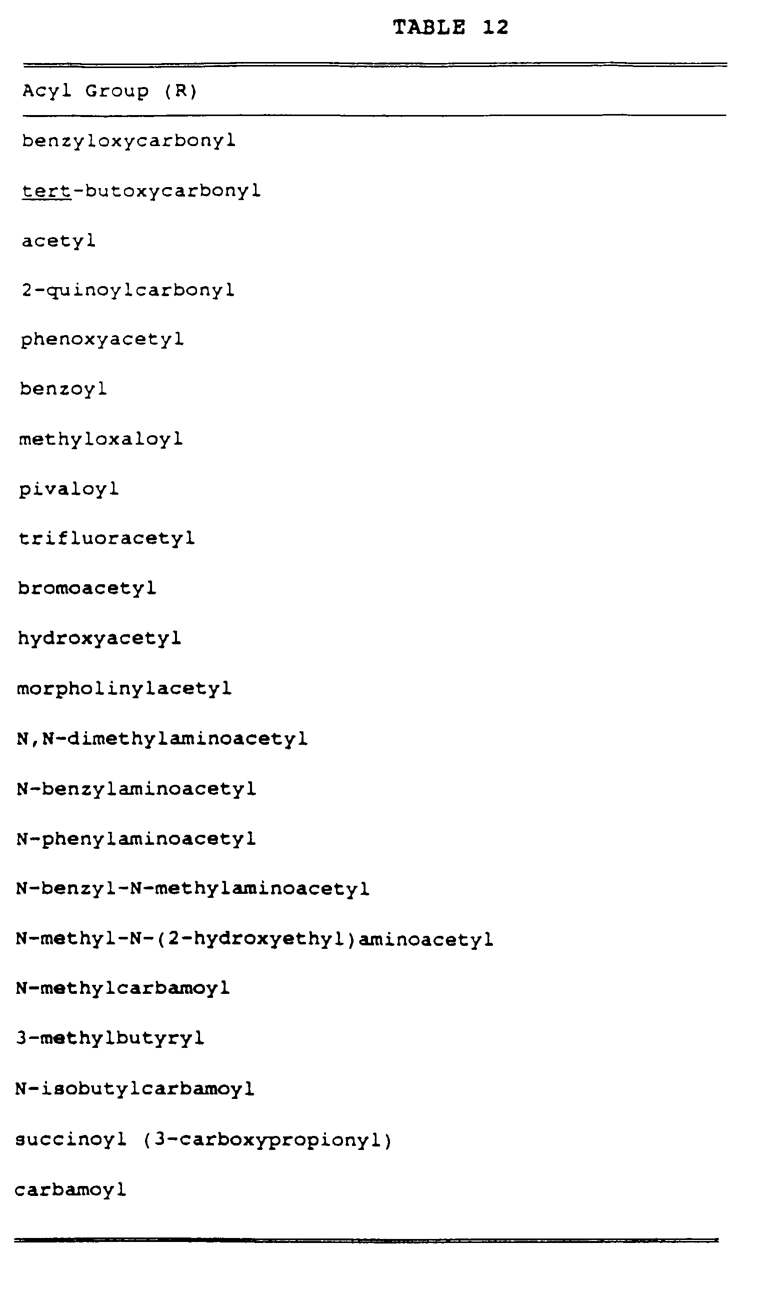

### Example 12

### Preparation of 3(S)-[N-(2-quinolinylcarbonyl)-L-asparaginyl]amino-2(R)-hydroxy-4-phenylbutylamine, N-(3-methylbutyl).

### Part A:

Preparation of N-3(S)-(Benzyloxycarbonyl)-amino-2(R)-hydroxy-4-phenylbutylamine, N-(3-methylbutyl). A solution of 20g (67 mmol) of N-benzyloxycarbonyl-3(S)-amino-1,2-(S)-epoxy-4-phenylbutane in 140 mL of isopropyl alcohol was treated with 83g (952 mmol) of isoamylamine and refluxed for one hour. The solution was cooled, concentrated, hexane added and the resulting solid filtered to afford 22.4g of the desired product.

### Part B:

Preparation of N-3(S)-(Benzyloxycarbonyl)-amino-2(R)-hydroxy-4-phenylbutylamine, N-(3-methylbutyl)-N-(t-butyloxycarbonyl). To a solution of 22.4g (58.3 mmol) of product from Part A above, 6.48g (64.1 mmol) of triethylamine and 150 mg of N,N-dimethyl-4-aminopyridine in 200 mL of tetrahydrofuran at 0°C was added 12.7g (58.3 mmol) of di-t-butylpyrocarbonate in 10 mL of THF. After 3.5 hours at room temperature, the volatiles were removed, ethyl acetate added and washed with 5% citric acid, sat d NaHCO₃, dried and concentrated to afford 30g of crude product. Chromatography on silica gel using 20% ethyl acetate/hexane afforded 22.5g (79%) of the desired product.

### Part C:

Preparation of N-3(S)-[N-benzyloxycarbonyl-L-asparaginyl]amino-2(R)-hydroxy-4-phenylbutylamine, N-(3-methylbutyl)-N-(t-butyloxycarbonyl). A solution of 22.5g of product from Part B above in 200 mL of ethanol was hydrogenated over 5.9g of 10% palladium-on-carbon under 50 psig hydrogen for one hour. The catalyst was filtered and the solvent removed under reduced pressureto afford 15.7g of free amine. This was dissolved in 130 mL of DMF and 4.54g (44.9 mmol) of N-methylmorpholine an added to a mixture of 13.3g (49.9 mmol) N-benzyloxy-carbonyl-L-asparagine, 11.5g (74.9 mmol) of N-hydroxybenzotriazole and 10.5g (54.9 mmol) of EDCl in 120 mL of DMF at 0°C, which had been preactivated for one hour prior to the addition. The mixture was stirred for 2 hours at 0°C and then for 12 hours at room temperature. The reaction was poured into 1L of sat d aqueous sodium bicarbonate, the solid collected, dissolved in ethyl acetate, washed with water, sat d sodium bicarbonate, 5% citric acid and brine, dried and concentrated to afford 16.7g of the desired product.

### Part D:

Preparation of N-3(S)-[N-(2-quinolinylcarbonyl)-L-asparaginyl]amino-2(R)-hydroxy-4-phenylbutylamine, N-(3-methylbutyl)-N-(t-butyloxycarbonyl). A solution of 16.7g (28.0 mmol) of product from Part C in 250 mL of methanol was hydrogenated over 6.0g of 10% palladium-on-carbon and under 50 psig hydrogen for one hour. The catalyst was filtered and the solution concentrated to afford 10.0g of free amine. This was dissolved in 100 mL of methylene chloride, 4.35g (43 mmol) of N-methylmorpholine was added followed by 5.53g (20.5 mmol) of quinoline-2-carboxylic acid, N-hydroxysuccinimide ester. This was stirred at room temperature overnight, the solvent removed, ethyl acetate added and washed with 5% citric acid, sat d sodium bicarbonate, brine, dried and concentrated to afford 14g of crude product. Recrystallization from ethyl acetate and hexane afforded 10.5g (83%) of desired product.

### Part E:

Preparation of N-3(S)-[N-(2-quinolinylcarbonyl)-L-asparaginyl]amino-2(R)-hydroxy-4-phenylbutylamine, N-(3-methylbutyl). To 80 mL of 4N hydrochloric acid in dioxane was added 9.17g (14.8 mmol) of product from Part D above. After one hour, the product becomes gummy. The solvents were removed, diethyl ether added and removed and the residue dissolved in 20 mL of methanol. This solution was added to 400 mL of sat d aqueous sodium bicarbonate, the solids collected, washed with acetone and hexane and dried in vacuo over P₂O₅ to afford 4.75g of the desired product.

### Example 13

This example illustrates the procedure utilized to prepare compounds wherein the stereochemistry about the hydroxyl group is (S).

### Part A:

A solution of 3(S)-(1,1-dimethylethoxycarbonyl)amino-1,2-(R)-epoxy-4-phenylbutane (1.00g, 3.80 mmol) and isobutylamine (5.55g, 76 mmol, 20 equiv.) in 10 mL of isopropyl alcohol was warmed to 60°C for 1 hour. The solution was cooled to room temperature and concentrated *in vacuo* and the residue recrystallized from hexane/methylene chloride to give 0.93g, 73% of [2(S), 3(S)]-N-[[[3-[(1,1-dimethylethyl)carbamoyl]amino]]-2-hydroxy-4-phenylbutyl]N-[(3-methylbutyl)]amine, mp 91.3 - 93.0°C.

### Part B:

The product from Part A (46.3mg, 0.14 mmol) was dissolved in a mixture of 5 mL of tetrahydrofuran and 2 mL of methylene chloride and treated with tert-butylisocyanate (136.4mg, 1.376 mmol) via syringe. The solution was stirred at room temperature for 0.5 hour and then the solvent was removed *in vacuo.* The product, TLC on SiO₂, 1:1 hexane: Ethyl acetate had Rf = 0.74 and was used directly in the next step without further purification.

### Part C:

The crude product from Part B was taken up in 10 mL of 4N hydrochloric acid in dioxane and stirred at room temperature for 0.25 hours. The solvent and excess hydrochloric acid was removed *in vacuo* whereupon the product crystallized. The solid was isolated by filtration washed with acetone and dried *in vacuo* to 3-[[(1,1-dimethylethyl)amino]carbonyl](2-methylpropyl)amino-2(S)-hydroxy-1(S)-(phenylmethyl)propylamine hydrochloride.

### Part D:

A solution of N-Cbz-L-asparagine (225.5mg, 0.847 mmol) and N-hydroxybenzotriazole (182.9mg, 1.21 mmol) was dissolved in 2 mL of dimethylformamide and cooled to 0°C and then treated with EDC (170.2mg, 0.898 mmol) for 10 minutes. This mixture was then treated with 3-[[(1,1-dimethylethyl)amino]carbonyl](2-methylpropyl)amino-2(S)-hydroxy-1(S)-(phenylmethyl)propylamine hydrochloride. (300.Omg, 0.807 mmol) followed by N-methylmorpholine (90.Omg, 0.888 mmol) via syringe. The solution was stirred at room temperature for 16 hours and then poured into 20 mL of rapidly stirring 60% saturated aqueous sodium bicarbonate solution whereupon a white precipitate formed. The solid was isolated by filtration, washed with saturated aqueous sodium bicarbonate solution, water, 5% aqueous citric acid solution, water and then dried *in vacuo* to give 319mg, 68% of butanediamide, N¹-[3-[[[(1,1-dimethylethyl)amino]carboyl](2-methylpropyl)amino]-2(S)-hydroxy-1(S)-(phenylmethyl)propyl]-2(S)-[(benzyloxycarbonyl)amino] mp 139-141°C, MH⁺ m/z = 584.

### EXAMPLE 14

Following the above general procedures but subsituting an acid chloride or anhydride for the isocyanate or similar starting material, the compounds shown in Table 16 were prepared.

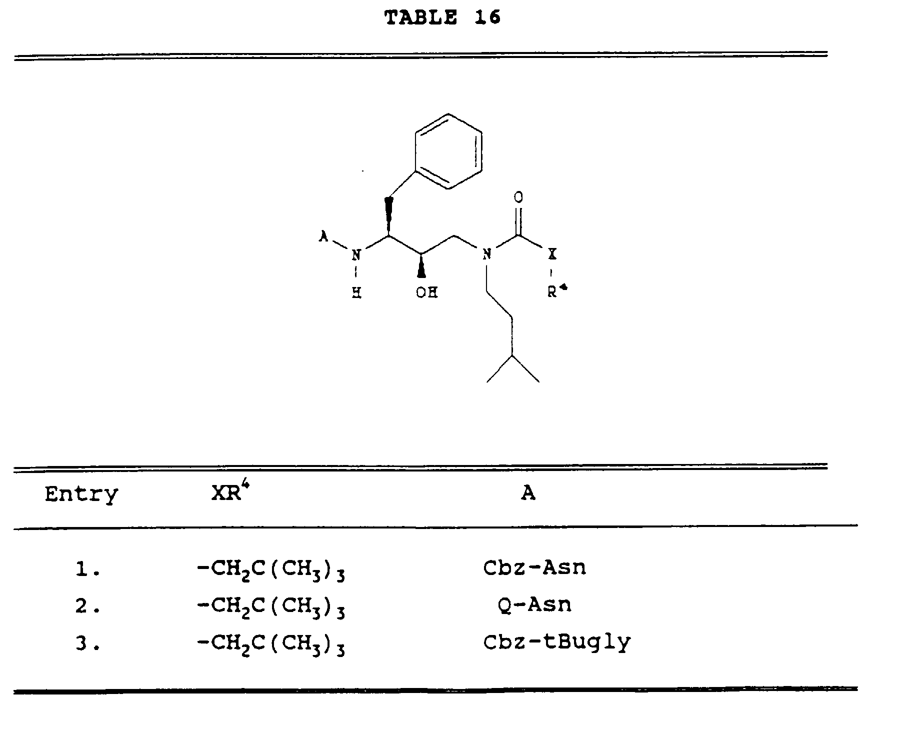

### EXAMPLE 15

Following the above general procedures but substituting a chloroformate or pyrocarbonate for the isocyante or similar starting material, the compounds shown in Table 17 were prepared.

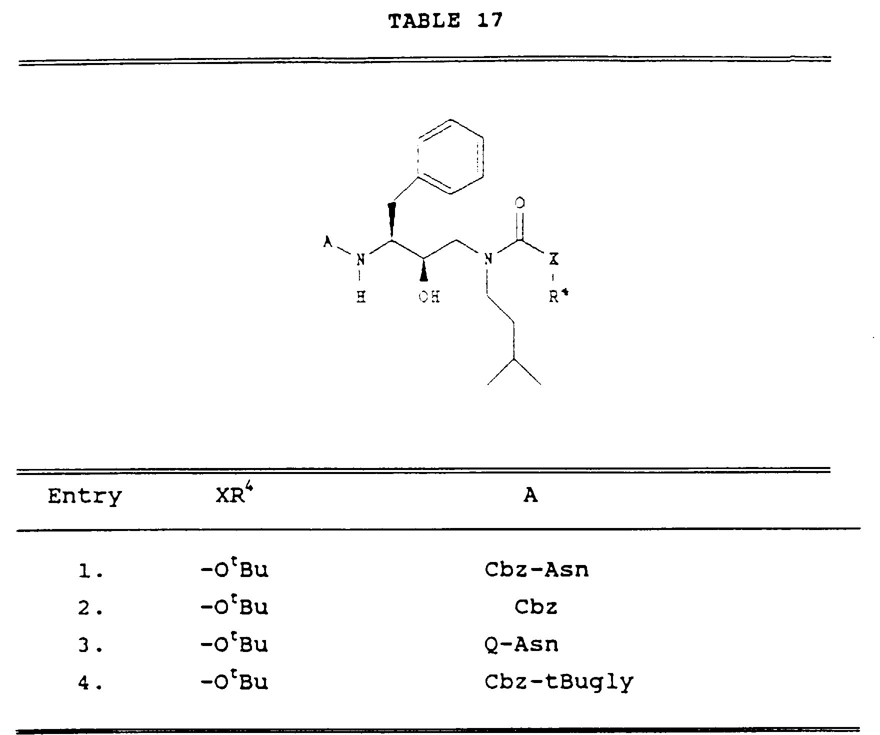

### EXAMPLE 16

The compounds of the present invention are effective HIV protease inhibitors. Utilizing an enzyme assay as described below, the compounds set forth in the examples herein disclosed inhibited the HIV enzyme. The preferred compounds of the present invention and their calculated IC₅₀ (inhibiting concentration 50%, i.e., the concentration at which the inhibitor compound reduces enzyme activity by 50%) values are shown in Table 18. The enzyme method is described below. The substrate is 2-aminobenzoyl-Ile-Nle-Phe(p-NO₂)-Gln-ArgNH₂. The positive control is MVT-101 (Miller, M. et al, Science, 246, 1149 (1989)] The assay conditions are as follows: Assay buffer:
20 mM sodium phosphate, pH 6.4
20% glycerol
1 mM EDTA
1 mM DTT
0.1% CHAPS

The above described substrate is dissolved in DMSO, then diluted 10 fold in assay buffer. Final substrate concentration in the assay is 80 µM.

HIV protease is diluted in the assay buffer to a final enzyme concentration of 12.3 nanomolar, based on a molecular weight of 10,780.

The final concentration of DMSO is 14% and the final concentration of glycerol is 18%. The test compound is dissolved in DMSO and diluted in DMSO to 10x the test concentration; 10µl of the enzyme preparation is added, the materials mixed and then the mixture is incubated at ambient temperature for 15 minutes. The enzyme reaction is initiated by the addition of 40µl of substrate. The increase in fluorescence is monitored at 4 time points (0, 8, 16 and 24 minutes) at ambient temperature. Each assay is carried out in duplicate wells.

**TABLE 18**

| | Compound | IC₅₀(nanomolar) |
|---|---|---|
| 1. | Butanediamide, N¹-[3-[[[(2,2-dimethyl)propyl]carbonyl](3-methylbutyl)amino]-2-hydroxy-1-(phenylmethyl)propyl]-2-[(2-quinolinylcarbonyl)amino]-, [1S-[1R*(2R*),2S*]] | 21nM |
| 2. | Butanediamide, N¹-[3-[[butylcarbonyl](cyclohexylmethyl)amino]-2-hydroxy-1-(phenylmethyl) propyl]-2-[(phenylmethyloxy)carbonyl)amino]-, [1S-[1R*(2R*),2S*]] | 696nM |
| 3. | Carbamic acid, [3-[[4-amino-1,4-dioxo-2-[(phenylmethyloxy)carbonyl]amino]butyl]amino]-2-hydroxy-4-phenylbutyl](phenylmethyl)-, butyl ester, [2R- [2R*,3S*(S*)]] | 1.6mM |

### Example 17

The effectiveness of the compounds listed in Table 19 were determined in the above-described enzyme assay and in a CEM cell assay.

The HIV inhibition assay method of acutely infected cells is an automated tetrazolium based colorimetric assay essentially that reported by Pauwles et al, J. Virol. Methods 20, 309-321 (1988). Assays were performed in 96-well tissue culture plates. CEM cells, a CD4⁺ cell line, were grown in RPMI-1640 medium (Gibco) supplemented with a 10% fetal calf serum and were then treated with polybrene (2µg/ml). An 80 µl volume of medium containing 1 x 10⁴ cells was dispensed into each well of the tissue culture plate. To each well was added a 100µl volume of test compound dissolved in tissue culture medium (or medium without test compound as a control) to achieve the desired final concentration and the cells were incubated at 37°C for 1 hour. A frozen culture of HIV-1 was diluted in culture medium to a concentration of 5 x 10⁴ TCID₅₀ per ml (TCID₅₀ = the dose of virus that infects 50% of cells in tissue culture), and a 20µL volume of the virus sample (containing 1000 TCID₅₀ of virus) was added to wells containing test compound and to wells containing only medium (infected control cells). Several wells received culture medium without virus (uninfected control cells). Likewise, the intrinsic toxicity of the test compound was determined by adding medium without virus to several wells containing test compound. In summary, the tissue culture plates contained the following experiments:

| | Cells | Drug | Virus |
|---|---|---|---|
| 1. | + | - | - |
| 2. | + | + | - |
| 3. | + | - | + |
| 4. | + | + | + |

In experiments 2 and 4 the final concentrations of test compounds were 1, 10, 100 and 500 µg/ml. Either azidothymidine (AZT) or dideoxyinosine (ddI) was included as a positive drug control. Test compounds were dissolved in DMSO and diluted into tissue culture medium so that the final DMSO concentration did not exceed 1.5% in any case. DMSO was added to all control wells at an appropriate concentration.

Following the addition of virus, cells were incubated at 37°C in a humidified, 5% CO₂ atmosphere for 7 days. Test compounds could be added on days 0, 2 and 5 if desired. On day 7, post-infection, the cells in each well were resuspended and a 100µl sample of each cell suspension was removed for assay. A 20µL volume of a 5 mg/ml solution of 3-(4,5-dimethylthiazol-2-yl)-2,5-diphenyltetrazolium bromide (MTT) was added to each 100µL cell suspension, and the cells were incubated for 4 hours at 27°C in a 5% CO₂ environment. During this incubation, MTT is metabolically reduced by living cells resulting in the production in the cell of a colored formazan product. To each sample was added 100µl of 10% sodium dodecylsulfate in 0.01 N HCl to lyse the cells, and samples were incubated overnight. The absorbance at 590 nm was determined for each sample using a Molecular Devices microplate reader. Absorbance values for each set of wells is compared to assess viral control infection, uninfected control cell response as well as test compound by cytotoxicity and antiviral efficacy.

**TABLE 19**

| Compound | Inhibition |
|---|---|
| 1. Butanediamide, N¹-[3-[[[(2,2-dimethyl)propyl]carbonyl](3-methylbutyl)amino]-2-hydroxy-1-(phenylmethyl)propyl]-2-[(2-quinolinylcarbonyl)amino]-, [1S-[1R*(2R*),2S*]] | 100% |

Following the procedures set forth above, the following compounds were also prepared:
Carbamic acid, [3-[[(4-amino-1,4-dioxo-2-[(2-quinolinylcarbonyl)amino]butyl]amino]-2-hydroxy-4-phenylbutyl][(4-fluorophenyl)methyl]-,1,1-dimethylethyl ester, [2R-[2R*,3S*(S*)]]-
Carbamic acid, [3-[[(4-amino-1,4-dioxo-2-[(2-quinolinylcarbonyl)amino]butyl]amino]-2-hydroxy-4-phenylbutyl][(3-methylbutyl)]-,1,1-dimethylethyl ester, [2R-[2R*,3S*(S*)]]-
Carbamic acid, [3-[[(4-amino-1,4-dioxo-2-[(2-quinolinylcarbonyl)amino]butyl]amino]-2-hydroxy-4-phenylbutyl][(2-methylpropyl)]-,1,1-dimethylethyl ester, [2R-[2R*,3S*(S*)]]-
Carbamic acid, [3-[[(4-amino-1,4-dioxo-2-[(2-quinolinylcarbonyl)amino]butyl]amino]-2-hydroxy-4-phenylbutyl][(4-pyridylmethyl)]-,1,1-dimethylethyl ester, [2R-[2R*,3S*(S*)]]-

The compounds of the present invention are effective antiviral compounds and, in particular, are effective retroviral inhibitors as shown above. Thus, the subject compounds are effective HIV protease inhibitors. It is contemplated that the subject compounds will also inhibit other viruses such as HIV, human T-cell leukemia virus, respiratory syncitial virus, hepadnavirus, cytomegalovirus and picornavirus.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. When administered as a combination, the therapeutic agents can be formulated as separate compositions which are given at the same time or different times, or the therapeutic agents can be given as a single composition.

The preceding examples can be repeated with similar success by substituting the generically or specifically described reactants and/or operating conditions of this invention for those used in the preceding examples.

## Claims

1. Compound represented by the formula: 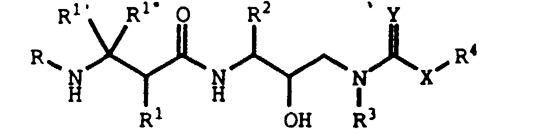 or a pharmaceutically acceptable salt thereof, wherein the stereochemistry about the hydroxy group is (R);
Y represents O or S;
R² represents alkyl, cycloalkylalkyl or aralkyl radicals, which radicals are optionally substituted with halogen, -OR⁹ or -SR⁹ radicals, wherein R⁹ represents alkyl radical; and
R¹ represents methyl radical and R^{1'} and R^{1"} independently represent hydrogen or radical as defined for R¹; and R represents alkanoyl, arylalkanoyl, aryloxyalkanoyl, arylalkoxycarbonyl, heteroaroyl or alkylaminocarbonyl radicals; or
R¹ and R^{1'} are both hydrogen and R^{1"} represents -CH₂SO₂NH₂, alkyl of 1 to 4 carbon atoms or cycloalkyl radicals or side chain of the amino acid asparagine, S-methyl cysteine or the sulfone or sulfoxide derivatives thereof, histidine, norleucine, glutamine, glycine, allo-isoleucine, alanine, threonine, isoleucine, leucine, tert-leucine, phenylalanine, ornithine, allo-threonine or valine; and R represents arylalkoxycarbonyl or heteroaroyl radicals; and
X represents O or C(R⁵)(R¹⁷), wherein R¹⁷ represents hydrogen or alkyl radicals; and
R³ and R⁴ independently represent alkyl, cycloalkyl, cycloalkylalkyl, aryl or aralkyl radicals; and R⁵ represents hydrogen radical; or
R³ represents alkyl radical of 2 to 5 carbon atoms; and R⁴ and R⁵ together with the carbon atom to which they are attached represent a 5 or 6-membered cycloalkyl radical optionally substituted with an alkyl radical of 1 to 3 carbon atoms; and
wherein alkyl, alone or in combination, is a straight-chain or branched-chain alkyl radical containing from 1 to 8 carbon atoms; cycloalkyl, alone or in combination, is an cyclic alkyl radical containing from 3 to 8 carbon atoms; aryl, alone or in combination, is an unsubstituted phenyl or naphthyl radical, or phenyl or naphthyl radical substituted with a C₁-C₈ alkyl, C₁-C₈ alkoxy, halogen, hydroxy or amino radical; heterocycloalkyl, alone or in combination, is pyrrolidinyl, piperidinyl, piperazinyl, morpholinyl, thiamorpholinyl, tetrahydroquinolinyl or tetrahydroisoquinolinyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido; and heteroaryl, alone or in combination, is pyrrolyl, imidazolyl, pyrazolyl, pyridyl, pyrazinyl, pyrimidinyl, furyl, thienyl, triazolyl, oxazolyl, thiazolyl, indolyl, quinolinyl, isoquinolinyl, quinoxalinyl, β-carbolinyl, benzofuranyl or benzimidazolyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁C₈-alkoxycarbonyl, C₁-C₈ alkanoyl, phenyl or phenyl-C₁-C₈-alkyl, or on a ring tertiary nitrogen atom by oxido.

2. Compound of Claim 1 wherein
R¹ represents methyl radical and R^{1'} and R^{1"} independently represent hydrogen or radical as defined for R¹; and R represents alkanoyl, arylalkanoyl, aryloxyalkanoyl, arylalkoxycarbonyl, heteroaroyl or alkylaminocarbonyl radicals; or
R¹ and R^{1'} are both hydrogen and R^{1"} represents alkyl of 1 to 4 carbon atoms; and R represents arylalkoxycarbonyl or heteroaroyl radicals; and
R³ and R⁴ independently represent alkyl, cycloalkyl or cycloalkylalkyl radicals; and R⁵ represents hydrogen radical; or
R³ represents alkyl radical of 2 to 5 carbon atoms; and R⁴ and R⁵ together with the carbon atom to which they are attached represent a 5 or 6-membered cycloalkyl radical.

3. Compound of Claim 2 wherein
R¹ represents methyl radical and R^{1'} and R^{1"} independently represent hydrogen or radical as defined for R¹; and R represents acetyl, phenoxyacetyl, 2-naphthyloxyacetyl, benzyloxycarbonyl, p-methoxybenzyloxycarbonyl, quinolinylcarbonyl, 2-benzofurancarbonyl or N-methylaminocarbonyl radicals; or
R¹ and R^{1'} are both hydrogen and R^{1"} represents alkyl radical of 1 to 4 carbon atoms; and R represents quinolinylcarbonyl or 2-benzofurancarbonyl radicals; and
R² represents CH₃SCH₂CH₂-, iso-butyl, n-butyl, benzyl, 4-fluorobenzyl, 2-naphthylmethyl or cyclohexylmethyl radicals;
R³ represents n-pentyl, n-hexyl, n-propyl, i-butyl, neopentyl, i-amyl, n-butyl, cyclohexylmethyl, benzyl, para-fluorobenzyl, para-methoxybenzyl, para-methylbenzyl or 2-naphthylmethyl radicals;
R⁴ represents t-butyl radical; and R⁵ is hydrogen radical.

4. A pharmaceutical composition comprising a compound according to any of claims 1 to 3 and a pharmaceutically acceptable carrier.

5. Use of a compound defined in any of claims 1 to 3 for the manufacture of a medicament for inhibiting retroviral protease.

6. Use according to claim 5 wherein the retroviral protease is HIV protease.

7. Use of a compound defined in any of claims 1 to 3 for the manufacture of a medicament for treatment of a retroviral infection.

8. Use according to claim 7 wherein the retroviral infection is an HIV infection.

9. Use of a compound defined in any of claims 1 to 3 for the manufacture of a medicament for the treatment of AIDS.

## Patentansprüche

1. Verbindung, dargestellt durch die Formel 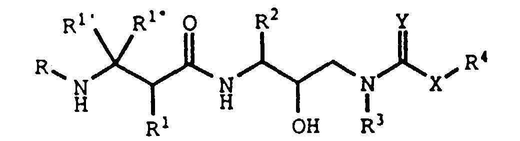 oder ein pharmazeutisch annehmbares Salz davon, worin die Stereochemie um die Hydroxygruppe (R) ist;
Y für O oder S steht;
R² Alkyl-, Cycloalkylalkyl- oder Aralkylreste darstellt, welche Reste gegebenenfalls mit Halogen-, -OR⁹- oder SR⁹-Resten substituiert sind, worin R⁹ einen Alkylrest darstellt; und
R¹ einen Methylrest darstellt und R^{1'} und R^{1*} unabhängig Wasserstoff oder einen Rest, wie für R¹ definiert darstellen; und R Alkanoyl-, Arylalkanoyl-, Aryloxyalkanoyl-, Arylalkoxycarbonyl-, Heteroaroyl- oder Alkylaminocarbonylreste darstellt; oder
R¹ und R¹, beide für Wasserstoff stehen und R^{1*} für CH₂SO₂NH₂-, Alkyl- mit 1 bis 4 Kohlenstoffatomen oder Cycloalkylreste oder die Seitenkette der Aminosäure Asparagin, S-Methylcystein oder den Sulfon- oder Sulfoxid-Derivaten davon, Histidin, Norleucin, Glutamin, Glycin, Allo-isoleucin, Alanin, Threonin, Isoleucin, Leucin, tert.-Leucin, Phenylalanin, Ornithin, Allo-threonin oder Valin steht; und R Arylalkoxycarbonyl- oder Heteroaroylreste darstellt; und
X für O oder C(R⁵)(R¹⁷) steht, worin R¹⁷ Wasserstoff oder Alkylreste darstellt; und
R³ und R⁴ unabhängig Alkyl-, Cycloalkyl-, Cycloalkylalkyl-, Aryl- oder Aralkylreste darstellen; und R⁵ einen Wasserstoffrest darstellt; oder
R³ einen Alkylrest mit 2 bis 5 Kohlenstoffatomen darstellt und R⁴ und R⁵ zusammen mit dem Kohlenstoffatom, an das sie gebunden sind, einen 5- oder 6-gliedrigen Cycloalkylrest darstellen, gegebenenfalls substituiert mit einem Alkylrest mit 1 bis 3 Kohlenstoffatomen; und
worin Alkyl allein oder in Kombination für einen geradkettigen oder verzweigtkettigen Alkylrest steht, der 1 bis 8 Kohlenstoffatome enthält; Cycloalkyl allein oder in Kombination für einen cyclischen Alkylrest steht, der 3 bis 8 Kohlenstoffatome enthält; Aryl allein oder in Kombination für einen nicht substituierten Phenyl- oder Naphthylrest, oder Phenyl- oder Naphthylrest, substituiert mit einem C₁-C₈-Alkyl-, C₁-C₈-Alkoxy-, Halogen-, Hydroxy- oder Aminorest steht; Heterocycloalkyl allein oder in Kombination für Pyrrolidinyl-, Piperidinyl-, Piperazinyl-, Morpholinyl-, Thiamorpholinyl-, Tetrahydrochinolinyloder Tetrahydroisochinolinylreste steht, welche Reste gegebenenfalls an einem substituierbaren Kohlenstoffatom mit Halogen, C₁-C₈-Alkyl, C₁-C₈-Alkoxy oder Oxo, an einem sekundären Ring-Stickstoffatom mit C₁-C₈-Alkyl, Aryl-C₁-C₈-alkoxycarbonyl, C₁-C₈-Alkanoyl, Phenyl oder Phenyl-C₁-C₈-alkyl, oder an einem tertiären Ring-Stickstoffatom durch Oxido substituiert sind; und Heteroaryl allein oder in Kombination für Pyrrolyl-, Imidazolyl-, Pyrazolyl-, Pyridyl-, Pyrazinyl-, Pyrimidiniyl-, Furyl-, Thienyl-, Triazolyl-, Oxazolyl-, Thiazolyl-, Indolyl-, Chinolinyl-, Isochinolinyl-, Chinoxalinyl-, β-Carbolinyl-, Benzofuranyl- oder Benzimidazolylreste steht, welche Reste gegebenenfalls an einem substituierbaren Kohlenstoffatom mit Halogen, C₁-C₈-Alkyl, C₁-C₈-Alkoxy oder Oxo, an einem sekundären Ring-Stickstoffatom mit C₁-C₈-Alkyl, Aryl-C₁-C₈-alkoxycarbonyl, C₁-C₈-Alkanoyl, Phenyl oder Phenyl-C₁-C₈-alkyl, oder an einem tertiären Ring-Stickstoffatom durch Oxido substituiert sind.

2. Verbindung von Anspruch 1, worin
R¹ einen Methylrest darstellt und R¹' und R^{1*} unabhängig Wasserstoff oder einen Rest, wie für R¹ definiert darstellen; und R für Alkanoyl-, Arylalkanoyl-, Aryloxyalkanoyl-, Arylalkoxycarbonyl-, Heteroaroyl- oder Alkylaminocarbonylreste steht; oder
R¹ und R^{1'} beide Wasserstoff darstellen und R^{1*} Alkyl mit 1 bis 4 Kohlenstoffatomen darstellt; und R Arylalkoxycarbonyloder Heteroaroylreste darstellt; und
R³ und R⁴ unabhängig Alkyl-, Cycloalkyl- oder Cycloalkylalkylreste darstellen; und R⁵ einen Wasserstoffrest darstellt; oder
R³ einen Alkylrest mit 2 bis 5 Kohlenstoffatomen darstellt; und R⁴ und R⁵ zusammen mit dem Kohlenstoffatom, an das sie gebunden sind, einen 5- oder 6-gliedrigen Cycloalkylrest darstellen.

3. Verbindung von Anspruch 2, worin
R¹ einen Methylrest darstellt und R^{1`} und R^{1*} unabhängig Wasserstoff oder einen Rest, wie für R¹ definiert darstellen; und R Acetyl-, Phenoxyacetyl-, 2-Naphthyloxyacetyl-; Benzyloxycarbonyl-, p-Methoxybenzyloxycarbonyl-, Chinolinylcarbonyl-, 2-Benzofurancarbonyl- oder N-Methylaminocarbonylreste darstellen; oder
R¹ und R^{1'} beide Wasserstoff darstellen und R^{1*} einen Alkylrest mit 1 bis 4 Kohlenstoffatomen darstellt; und R Chinolinylcarbonyl- oder 2-Benzofurancarbonylreste darstellt; und
R² für CH₃SCH₂CH₂-, Isobutyl-, n-Butyl-, Benzyl-, 4-Fluorbenzyl-, 2-Naphthylmethyl- oder Cyclohexylmethylreste steht;
R³ für n-Pentyl-, n-Hexyl, n-Propyl-, i-Butyl-, Neopentyl-, i-Amyl-, n-Butyl-, Cyclohexylmethyl-, Benzyl-, Parafluorbenzyl-, Paramethoxybenzyl-, Paramethylbenzyl- oder 2-Naphthylmethylreste steht;
R⁴ einen t-Butylrest darstellt; und R⁵ einen Wasserstoffrest darstellt.

4. Pharmazeutische Zusammensetzung, umfassend eine Verbindung gemäss einem der Ansprüche 1 bis 3 und einen pharmazeutisch annehmbaren Träger.

5. Verwendung einer in einem der Ansprüche 1 bis 3 definierten Verbindung für die Herstellung einer Arznei zum Hemmen retroviraler Protease.

6. Verwendung gemäss Anspruch 5, worin die retrovirale Protease HIV-Protease ist.

7. Verwendung einer in einem der Ansprüche 1 bis 3 definierten Verbindung für die Herstellung einer Arznei zur Behandlung einer retroviralen Infektion.

8. Verwendung gemäss Anspruch 7, worin die retrovirale Infektion eine HIV-Infektion ist.

9. Verwendung einer in einem der Ansprüche 1 bis 3 definierten Verbindung für die Herstellung einer Arznei zur Behandlung von AIDS.

## Revendications

1. Composé représenté par la formule : 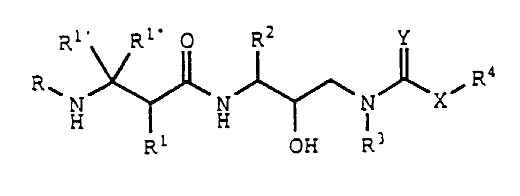 ou un sel pharmaceutiquement acceptable de celui-ci, où la stéréochimie en ce qui concerne le groupe hydroxy est (R) ;
Y représente O ou S ;
R² représente des radicaux alkyle, cycloalkylalkyle ou aralkyle, lesquels radicaux sont facultativement substitués par des atomes d'halogène, des radicaux -OR⁹ ou -SR⁹, où R⁹ représente un radical alkyle ; et
R¹ représente un radical méthyle et R^{1'} et R^{1"} représentent indépendamment un atome d'hydrogène ou un radical tel que défini pour R¹ ; et R représente des radicaux alcanoyle, arylalcanoyle, aryloxyalcanoyle, arylalcoxycarbonyle, hétéroaroyle ou alkylaminocarbonyle ; ou
R¹ et R^{1'} représentent tous les deux des atomes d'hydrogène et R^{1"} représente -CH₂SO₂NH₂, des radicaux alkyle comportant de 1 à 4 atomes de carbone, ou des radicaux cycloalkyle ou une chaîne latérale de l'amino-acide que constitue l'asparagine, la S-méthylcystéine ou les dérivés de type sulfone ou sulfoxyde de ceux-ci, l'histidine, la norleucine, la glutamine, la glycine, l'allo-isoleucine, l'alanine, la thréonine, l'isoleucine, la leucine, la tert-leucine, la phénylalanine, l'ornithine, l'allo-thréonine ou la valine ; et R représente des radicaux arylalcoxycarbonyle ou hétéroaroyle ; et
X représente O ou C(R⁵)(R¹⁷), où R¹⁷ représente un atome d'hydrogène ou des radicaux alkyle ; et
R³ et R⁴ représentent indépendamment des radicaux alkyle, cycloalkyle, cycloalkylalkyle, aryle ou aralkyle ; et R⁵ représente un atome d'hydrogène ; ou
R³ représente un radical alkyle comportant de 2 à 5 atomes de carbone ; et R⁴ et R⁵ représentent, ensemble avec l'atome de carbone auquel ils sont attachés, un radical cycloalkyle à 5 ou 6 chaînons facultativement substitué par un radical alkyle comportant de 1 à 3 atomes de carbone ; et
où un radical alkyle, seul ou en combinaison, est un radical alkyle à chaîne linéaire ou à chaîne ramifiée, contenant de 1 à 8 atomes de carbone ; un radical cycloalkyle, seul ou en combinaison, est un radical alkyle cyclique contenant de 3 à 8 atomes de carbone ; un radical aryle, seul ou en combinaison, est un radical phényle ou naphtyle non substitué, ou un radical phényle ou naphtyle substitué par un radical alkyle en C₁-C₈, alcoxy en C₁-C₈, un atome d'halogène, un radical hydroxy ou amino ; un radical hétérocycloalkyle, seul ou en combinaison, est un radical pyrrolidinyle, pipéridinyle, pipérazinyle, morpholinyle, thiamorpholinyle, tétrahydroquinolinyle ou tétrahydroisoquinolinyle, lesquels radicaux sont facultativement substitués, sur un atome de carbone pouvant être substitué, par un atome d'halogène, un radical alkyle en C₁-C₈, alcoxy en C₁-C₈, ou oxo, sur un atome d'azote secondaire du cycle par un radical alkyle en C₁-C₈, aryl(alcoxy en C₁-C₈)carbonyle, alcanoyle en C₁-C₈, phényle, ou phényl(alkyle en C₁-C₈), ou sur un atome d'azote tertiaire du cycle par un radical oxido ; et un radical hétéroaryle, seul ou en combinaison, est un radical pyrrolyle, imidazolyle, pyrazolyle, pyridyle, pyrazinyle, pyrimidinyle, furyle, thiényle, triazolyle, oxazolyle, thiazolyle, indolyle, quinolinyle, isoquinolinyle, quinoxalinyle, β-carbolinyle, benzofuranyle ou benzimidazolyle, lesquels radicaux sont facultativement substitués, sur un atome de carbone pouvant être substitué, par un atome d'halogène, un radical alkyle en C₁-C₈, alcoxy en C₁-C₈, ou oxo, sur un atome d'azote secondaire du cycle, par un radical alkyle en C₁-C₈, aryl-(alcoxy en C₁-C₈) carbonyle, alcanoyle en C₁-C₈, phényle, ou phényl(alkyle en C₁-C₈), ou sur un atome d'azote tertiaire du cycle par un radical oxido.

2. Composé selon la revendication 1, dans lequel R¹ représente un radical méthyle et R^{1'} et R^{1"} représentent indépendamment un atome d'hydrogène ou un radical tel que défini pour R¹ ; et R représente des radicaux alcanoyle, arylalcanoyle, aryloxyalcanoyle, arylalcoxycarbonyle, hétéroaryle ou alkylaminocarbonyle ; ou
R¹ et R^{1'} représentent tous les deux des atomes d'hydrogène, et R^{1"} représente un groupe alkyle comportant de 1 à 4 atomes de carbone ; et R représente des radicaux arylalcoxycarbonyle ou hétéroaroyle ; et
R³ et R⁴ représentent indépendamment des radicaux alkyle, cycloalkyle ou cycloalkylalkyle ; et R⁵ représente un atome d'hydrogène ; ou
R³ représente un radical alkyle comportant de 2 à 5 atomes de carbone ; et R⁴ et R⁵ représentent, ensemble avec l'atome de carbone auquel ils sont attachés, un radical cycloalkyle à 5 ou 6 chaînons.

3. Composé selon la revendication 2, dans lequel
R¹ représente le radical méthyle et R^{1'} et R^{1"} représentent indépendamment un atome d'hydrogène ou un radical tel que défini pour R¹ ; et R représente des radicaux acétyle, phénoxyacétyle, 2-naphtyloxyacétyle, benzyloxycarbonyle, p-méthoxybenzyloxycarbonyle, quinolinylcarbonyle, 2-benzofuranecarbonyle ou N-méthylaminocarbonyle ; ou
R¹ et R^{1'} représentent tous les deux des atomes d'hydrogène et R^{1"} représente un radical alkyle comportant de 1 à 4 atomes de carbone ; et R représente des radicaux quinolinylcarbonyle ou 2-benzofuranecarbonyle ; et
R² représente CH₃SCH₂CH₂-, des radicaux iso-butyle, n-butyle, benzyle, 4-fluorobenzyle, 2-naphtylméthyle ou cyclohexylméthyle ;
R³ représente des radicaux n-pentyle, n-hexyle, n-propyle, i-butyle, néo-pentyle, i-amyle, n-butyle, cyclohexylméthyle, benzyle, parafluorobenzyle, para-méthoxybenzyle, para-méthylbenzyle ou 2-naphtylméthyle ;
R⁴ représente le radical t-butyle ; et R⁵ représente un atome d'hydrogène.

4. Composition pharmaceutique comprenant un composé selon l'une quelconque des revendications 1 à 3, et un support pharmaceutiquement acceptable.

5. Utilisation d'un composé selon l'une quelconque des revendications 1 à 3, pour la fabrication d'un médicament pour inhiber une protéase rétrovirale.

6. Utilisation selon la revendication 5, dans laquelle la protéase rétrovirale est la protéase du HIV.

7. Utilisation d'un composé selon l'une quelconque des revendications 1 à 3, pour la fabrication d'un médicament pour le traitement d'une infection rétrovirale.

8. Utilisation selon la revendication 7, dans laquelle l'infection rétrovirale est une infection par le HIV.

9. Utilisation d'un composé selon l'une quelconque des revendications 1 à 3, pour la fabrication d'un médicament pour le traitement du SIDA.
